(19)

Europäisches
Patentamt
European
Patent Office
Office européen
des brevets

(11) **EP 2 804 121 A2**

(12) **EUROPEAN PATENT APPLICATION**

(43) Date of publication:
**19.11.2014 Bulletin 2014/47**

(51) Int Cl.:
***G06F 19/00*** *(2011.01)*

(21) Application number: **14168542.0**

(22) Date of filing: **15.05.2014**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **17.05.2013 JP 2013104659**

(71) Applicant: **Hitachi Ltd.
Tokyo (JP)**

(72) Inventors:
• **Miyoshi, Toshinori
Chiyoda-ku, Tokyo 100-8280 (JP)**

• **Hasegawa, Yasutaka
Chiyoda-ku, Tokyo 100-8280 (JP)**
• **Ban, Hideyuki
Chiyoda-ku, Tokyo 100-8280 (JP)**
• **Nagasaki, Takeshi
Chiyoda-ku, Tokyo 100-8280 (JP)**
• **Shinjo, Hiroshi
Chiyoda-ku, Tokyo 100-8280 (JP)**

(74) Representative: **Moore, Graeme Patrick et al
Mewburn Ellis LLP
33 Gutter Lane
London
EC2V 8AS (GB)**

(54) **Analysis system and health business support method**

(57) It is provided an analysis system comprising a processor executing a program and a memory storing the program. The analysis system further comprises a data mapping unit controlling the processor to set an attractive force and a repulsive force acting between the instances based on the similarity information between data, and arranging the instances in a vector space having a certain number of dimensions so that an energy caused by the attractive force and the repulsive force is less than a threshold defined in advance, and a clustering unit classifying the instances arranged in the vector space. The data mapping unit is configured to virtually add one dimension to the vector space, add a minute fluctuation to coordinates of the instances in a direction of the added dimension, and calculate a minimum number of dimensions of the vector space where the instances are stable with respect to the minute fluctuation.

Fig. 1

Printed by Jouve, 75001 PARIS (FR)

**Description**

CLAIM OF PRIORITY

The present application claims priority from Japanese patent application JP 2013-104659 filed on May 17, 2013, the content of which is hereby incorporated by reference into this application.

BACKGROUND OF THE INVENTION

This invention relates to a data analysis technology, and more particularly, to a system for analyzing medical data, to thereby support a healthcare business.

A health insurance society operates an insurance business, a healthcare guidance system, for providing healthcare guidances for preventing lifestyle diseases, and preventing severity thereof from increasing in order to reduce medical cost. However, resources such as public health nurses employed for the healthcare guidance and a cost for the healthcare guidances are limited. Therefore, a system for supporting an operation of effective and efficient insurance business is desired.

As a method for supporting the operation of the insurance business, in Japanese Patent Application Laid-open No. 2012-128670 A, there is described a healthcare business support system for selecting people subject to a healthcare guidance based on healthcare cost information, health checkup information, and healthcare guidance information. The healthcare business support system includes a medical cost model generation unit for generating a medical cost model representing an estimated medical cost for each of severities and test values of an insured person to a health insurance, a test value improvement model generation unit for generating a test value improvement model representing an improvement amount for each of the severities and the test values, an estimated medical cost reduction effect calculation unit for calculating an estimated medical cost reduction amount by a healthcare guidance for each of the severities and the test values, and a subject person selection unit for selecting an insured person to the health insurance belonging to a severity and a test value high in estimated medical cost reduction amount as a healthcare guidance subject person.

SUMMARY OF THE INVENTION

It is necessary to select people subject to the healthcare guidance by priority in order to effectively and efficiently operate the insurance business within resources of a health insurance society. Moreover, a content of the healthcare guidance appropriate for each of the subject people needs to be selected.

When the medical cost is estimated according to Japanese Patent Application Laid-open No. 2012-128670 A, the future medical cost is estimated based on the current severity and test value. For example, a future severity of diabetes is estimated based on current severity and blood sugar level of diabetes, and an average medical cost corresponding to the severity is considered as an estimated medical cost.

However, a factor (blood sugar level for diabetes) effective for estimating the future medical cost and severity needs to be manually set as prior knowledge. Moreover, definition of the severity also needs to be manually set.

Various factors such as age, sex, other test values, a prescription state of medicines, and life style are considered in addition to blood sugar level as the factors effective for estimating the future medical cost, and more precise estimation can thus be carried out by considering the factors. However, it is difficult to manually list up the factors. Moreover, the factors need to be set based on the prior knowledge for each disease. Therefore, it is difficult to analyze all diseases.

The representative one of inventions disclosed in this application is outlined as follows. There is provided an analysis system comprising a processor configured to execute a program and a memory configured to store the program. The analysis system executes the program to analyze data. The analysis system is capable of making access to a storage apparatus configured to store similarity information between data including a similarity between instances. The analysis system further comprises a data mapping unit configured to control the processor to set an attractive force and a repulsive force acting between the instances based on the similarity information between data, and arrange the instances in a vector space having a certain number of dimensions so that an energy caused by the attractive force and the repulsive force is less than a threshold defined in advance, and a clustering unit configured to classify the instances arranged in the vector space. The data mapping unit is further configured to virtually add one dimension to the vector space, add a minute fluctuation to coordinates of the instances in a direction of the added dimension, and calculate a minimum number of dimensions of the vector space where the instances are stable with respect to the minute fluctuation.

According to the representative embodiment of this invention, the instances can be arranged based on the similarity between instances in the vector space having an appropriate number of dimensions. Therefore, the instances can be appropriately classified by means of a related-art clustering method. Objects, configurations, and effects which have not been described become apparent from a description of the following embodiments.

BRIEF DESCRIPTION OF THE DRAWINGS

The present invention can be appreciated by the description which follows in conjunction with the following figures, wherein:

FIG. 1 is a block diagram illustrating a configuration of a medical data analysis system according to a second embodiment;
FIG. 2 is a block diagram illustrating a configuration of a data analysis apparatus according to a first embodiment;
FIG. 3 is an explanatory diagram illustrating similarity information between data;
FIG. 4 is an explanatory diagram illustrating a related technology of this invention;
FIG. 5 is a flowchart of processing by a data mapping unit according to the first embodiment;
FIG. 6 is an explanatory diagram illustrating healthcare cost basic information according to the second embodiment;
FIG. 7 is an explanatory diagram illustrating health checkup information according to the second embodiment;
FIG. 8 is an explanatory diagram illustrating medical inquiry information according to the second embodiment;
FIG. 9 is an explanatory diagram illustrating injury and illness name information according to the second embodiment;
FIG. 10 is an explanatory diagram illustrating injury and illness name classification information according to the second embodiment;
FIG. 11 is an explanatory diagram illustrating clinical action information according to the second embodiment;
FIG. 12 is an explanatory diagram illustrating clinical action classification information according to the second embodiment;
FIG. 13 is an explanatory diagram illustrating medicine information according to the second embodiment;
FIG. 14 is an explanatory diagram illustrating medicine classification information according to the second embodiment;
FIG. 15 is an explanatory diagram illustrating an example of formatted information according to the second embodiment;
FIG. 16A is an explanatory diagram illustrating an example of formatted information according to the second embodiment;
FIG. 16B is a flowchart of item unification processing according to the second embodiment;
FIG. 17A is a flowchart of processing by a support function for a health insurance business operator according to the second embodiment;
FIG. 17B is a flowchart of processing by a support function for a responsible person and a subject person according to the second embodiment;
FIG. 18A is a flowchart of cluster characterization processing according to the second embodiment;
FIG. 18B is an explanatory diagram illustrating a display example of selected items and values according to the second embodiment; and
FIG. 19 is a flowchart of reconfiguration processing according to the second embodiment.

DETAILED DESCRIPTION OF THE PREFERRED EMBODIMENTS

[First embodiment]

In a first embodiment of this invention, a description is given of a data analysis apparatus for clustering data based on a similarity if similarity information between data describing a similarity between a pair of data is provided.

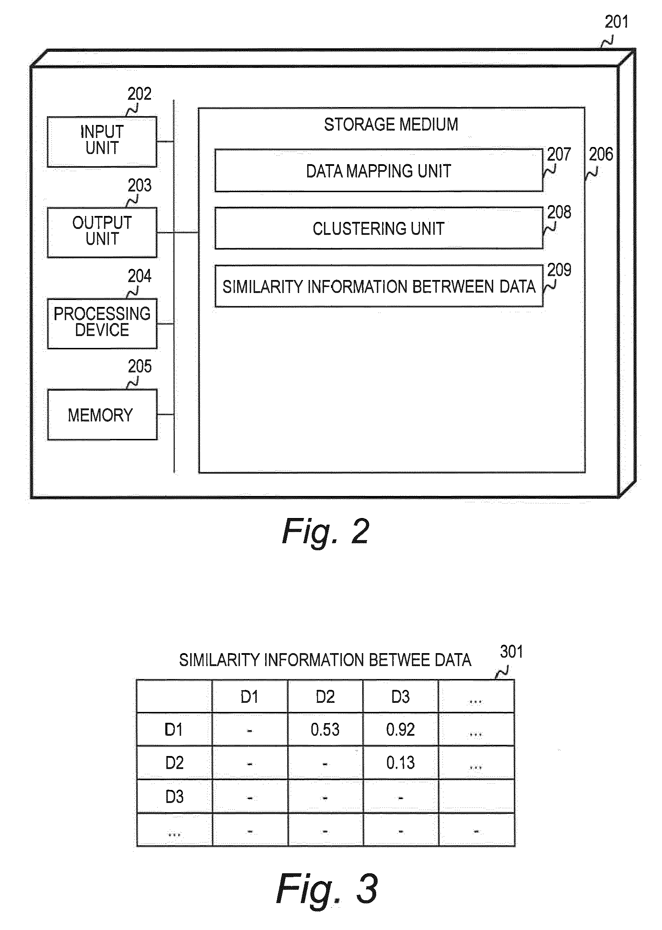

FIG. 2 is a block diagram illustrating a configuration of the data analysis apparatus according to the first embodiment.
A data analysis apparatus 201 includes an input unit 202, an output unit 203, a processing device 204, a memory 205, and a storage medium 206.
The input unit 202 is a human interface such as a mouse and a keyboard, and receives an input to the data analysis apparatus 201. The output unit 203 includes a display and a printer for outputting arithmetic operation results by a medical data analysis system. The storage medium 206 is a storage apparatus for storing various programs for realizing data analysis processing by the data analysis apparatus 201, execution results of the medical data analysis processing, and the like, and is, for example, a non-volatile storage medium (such as a magnetic disk drive and non-volatile memory). The processing device 204 is an arithmetic operation apparatus for executing a program loaded on the memory 205, and is, for example, a CPU, a GPU, or the like.
The data analysis apparatus 201 according to the first embodiment may be a computer system constituted by a single computer, or a computer system constituted by a server and client terminals. Moreover, the respective units of the data analysis apparatus 201 may be constituted by independent apparatus. The data analysis apparatus 201 is a computer system constituted on a single computer, or a plurality of logically or physically constituted computers, and may operate as independent threads on the same computer, or may operate on virtual computers constructed on a plurality of physical computer resources.

The program executed by the processing device 204 is provided for respective servers by means of a removable medium (such as a CD-ROM and a flash memory) or a network, and is stored in a non-volatile storage apparatus which is a non-transitory storage medium. Therefore, the computer system is preferred to include an interface for reading the removable medium.

A description is now given of processing carried out by the data analysis apparatus 201 according to the first embodiment.

First, a description is given of similarity information between data used in the first embodiment.

FIG. 3 is an explanatory diagram illustrating the similarity information between data.

Similarity information between data 301 describes similarities each between two different instances. For example, the similarity information between data 301 represents that the similarity between an instance D1 and an instance D3 is 0.92. The instance is, for example, "person". In this case, the similarity information between data 301 prescribes the similarity between two persons defined by a certain method (for example, a frequency of receiving/transmitting mails). Another example of the instance is "document". In this case, the similarity information between data 301 prescribes the similarity between documents defined by a certain method (such as a ratio of words appearing in both the documents out of all words appearing in the two documents). The data analysis apparatus 201 carries out the clustering of classifying instances into similar instances.

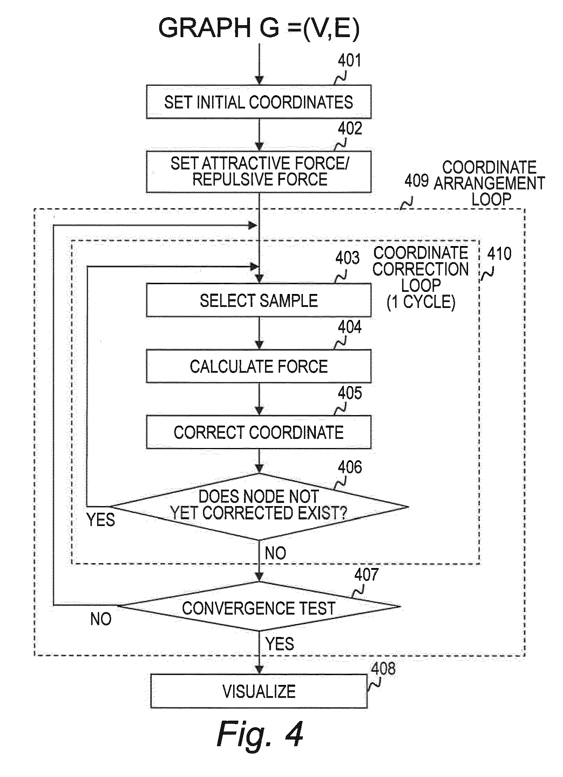

Referring to FIG. 4, a description is now given of a related technology of this invention, namely, a part of a technology described in Y. F. Hu, "Efficient, High-Quality Force-Directed Graph Drawing", The Mathematica Journal, vol. 10, no. 1, pp. 37-71, 2006, relating to this embodiment.

The technology described in "Efficient, High-Quality Force-Directed Graph Drawing" is a technology of visualizing a network in a two-dimensional space or a three-dimensional space. In the following, a network is a combination of a set V of nodes and a set E of edges, and is represented as a graph G=(V, E). A state where two of an $i$-th node and a $j$-th node are coupled to each other via an edge defined in the set E is represented as $i \Leftrightarrow j$.

A force-directed algorithm described below defines attractive force and repulsive force between nodes based on a presence/absence of an edge. Then, an arrangement of the nodes of the set V is corrected in a two-dimensional or three-dimensional space so that energies between the nodes decrease based on the defined attractive force and repulsive force. Thus, a stable arrangement of the nodes is defined.

A description is now given of definitions of the attractive force and the repulsive force acting between two nodes. The repulsive force $fr(i,j)$ and the attractive force $fa(i,j)$ acting between the $i$-th node and the $j$-th node are defined by Equations (1) and (2).

$$fr(i,j) = -CK^2 / |xi-xj| \quad i \neq j, \quad i,j \in V \ \dots \ (1)$$

$$fa(i,j) = |xi-xj|^2 / K, \text{ if } i \Leftrightarrow j \text{ holds true } \dots \ (2)$$

$fa(i,j)=0$, if $i \Leftrightarrow j$ does not hold true

In Equations (1) and (2), $xi$ and $xj$ are respectively coordinates of the $i$-th d $j$-th nodes in the two-dimensional or three-dimensional space, and $|xi-xj|$ is a Euclidean distance between $xi$ and $xj$. Moreover, C and K are positive constants, and $X^n$ represents an $n$-th power of $X$ for values $X$ and $n$. The repulsive force $fr$ is defined so as to act between all nodes, and the attractive force $fa$ is defined so as to act between nodes coupled to each other via an edge. The repulsive force between nodes may be defined by Equation (3) where p> 1.

$$fr(i,j) = -CK^{(1+p)} / |xi-xj|^p \quad i \neq j, \ i,j \in V \ \dots \ (3)$$

In an intuitive way, Equation (3) has such a meaning that an effectively acting extent of the repulsive force is adjusted, and the effectively acting extent of the repulsive force decreases as p increases.

A force $f(i)$ acting on the node $i$ is represented by summing the force defined as described above as Equation (4).

$$f(i)=-\Sigma fr(i,j)v(j,i)+\Sigma fa(i,j)v(j,i) \ldots (4)$$

In Equation (4), the sum is calculated for all nodes $j$ other than the node $i$. Moreover, $v(j,i)$ is a unit vector directing from xi to $xj$, and is defined by Equation (5).

$$v(j,i)=(xj-xi)/|xj-xi| \ldots (5)$$

An energy E of the entire system is defined by using Equation (5) as Equation (6).

$$E(\{xi\},K,C)=\Sigma|f(i)|^2 \ldots (6)$$

The sum is calculated for all nodes $i$, and $|f(i)|$ is a magnitude of a vector $f(i)$ in Equation (6). Moreover, $\{xi\}=\{x1, x2, \ldots \}$, and represents coordinate arrangement of all nodes.

An algorithm iteratively corrects the coordinates $\{xi\}$ of the nodes so as to reduce the energy E. As a result, a final arrangement of all the nodes is defined.

The constants $K$ and $C$ are logically constants relating to scaling of the coordinates in Equation (6), and do not essentially relate to the arrangement between the nodes. It is assumed that an optimal arrangement which minimizes the energy E where the constants $K$ and $C$ are used is $\{xi\}$. On this occasion, an optimum arrangement for minimizing the energy E when constants $K'$ and C' are used is defined as Equation (7) where $s=(K'/K)(C'/C)^{1/3}$.

$$\{xi\}=\{sx1,sx2, \ldots \} \ldots (7)$$

In Equation (7), $sxi$ is a vector generated by multiplying each of the elements of $xi$ by s. This is understood by recognizing that a relationship represented by Equation (8) holds true.

$$E(\{xi\},K,C)=(K/K')^2(C/C')^{4/3} E(\{sxi\},K',C') \ldots (8)$$

Referring to FIG. 4, a description is given of the algorithm of arranging the nodes in the two-dimensional or three-dimensional space by successively correcting the coordinate positions of the nodes while the attractive force and the repulsive force is calculated.

First, in Initial coordinate setting step 401, the number of dimensions is set to two or three, and an initial arrangement of the coordinates $\{xi\}$ of each node is defined in a vector space having the set number of dimensions. The initial arrangement of the coordinate $\{xi\}$ of the node may be set by using a random number, for example. Moreover, the nodes may be classified by means of a certain method, and nodes belonging to the same class may be arranged close to one another. Further, an arrangement defined by using another simple visualization algorithm may be the initial arrangement.

Then, in Attractive force/repulsive force setting step 402, attractive force is set between nodes having an edge, and repulsive force is set between all the nodes as described before. Predetermined values are used for the constants $C$ and $K$, the parameter $p$ of the repulsive force $fr$, and the like.

Then, in a coordinate arrangement loop 409, the coordinate arrangement $\{xi\}$ of each of the nodes is successively corrected so as to reduce the energy E. In other words, the coordinate arrangement loop 409 is a cycle of successively selecting each of the nodes from the graph G, and correcting the coordinate of the node. One cycle is a one round over the entire nodes.

A coordinate correction loop 410 is a cycle of successively selecting data, and correcting the coordinate of the data, and processing for one cycle is finished when the entire nodes are routed.

First, in Sample selection step 403, a node $i$ is selected from the entire nodes. Then, in Force calculation step 404, the sum $f(i)$ of the vectors of the force acting on the selected node $i$ is calculated. The sum $f(i)$ can be calculated by Equation (4).

Then, in Coordinate correction step 405, the coordinate $xi$ is corrected in a direction of the force acting on the selected node $i$ by using Equation (9).

$$xi \leftarrow xi + t \times f(i) / |f(i)| \quad \dots (9)$$

In Equation (9), $|f(i)|$ is the magnitude of $f(i)$, and $t$ is a parameter for adjusting a magnitude of the correction. The value of $t$ may initially be large for greatly correcting the coordinate, and may decrease for a final fine adjustment as the cycle repeats. For example, an initial value having an appropriate magnitude may be first set, and the value $t$ may be updated by using Equation (10) after the coordinate correction for one cycle is finished.

$$t \leftarrow 0.9 \times t \quad \dots (10)$$

Then, in Step 406, whether a node which has not been selected out of all the nodes exists or not is determined. As a result of the determination, if a node which has not been selected out of all the nodes exists, the algorithm returns to Sample selection step 403, and selects a next sample. On this occasion, the force acting from the node $i$ to other nodes have changed as a result of the correction of the coordinate $xi$, and, in Force calculation step 404, force vectors are calculated for the new coordinate arrangement by reflecting the correction of the coordinate. On the other hand, if all the nodes have been selected, the algorithm proceeds to Convergence determination step 407.

In Convergence determination step 407, a degree of convergence of the algorithm is determined, thereby determining whether the algorithm is finished or not. For example, a coordinate arrangement $\{yi\}$ of the node $i$ after one cycle of the coordinate correction for the last time and a coordinate arrangement $\{xi\}$ of the node $i$ after one cycle of the coordinate correction for this time are compared with each other, and if a correction amount is smaller than a predetermined threshold, the algorithm determines that the coordinates are sufficiently converged, and is finished. The correction amount may be calculated by using, for example, $\sum |xi-yi|$.

Then, in Visualization step 408, the nodes of the graph G are arranged on a screen for visualization based on the arrangement of the respective nodes in the two or three dimensional space.

The method described by referring to FIG. 4 is intended for the visualization, and the number of dimensions for the arrangement is first set to two or three. However, if a large number of nodes exist, it is difficult to represent the positional relationship among the nodes in the space having a low dimension such as two or three dimensions. The method illustrated in FIG. 4 can be easily extended to a space having four or more dimensions by arranging the nodes in a space having a large number of dimensions, and correcting the arrangement of the nodes. However, the method illustrated in FIG. 4 determines the number of dimensions of the space before the nodes are arranged, and it is difficult to select an appropriate number of dimensions for representing the positional relationship among data. For example, if the predetermined number of dimensions is small, the positional relationship among data cannot be sufficiently represented. On the other hand, if the predetermined number of dimensions is large, the representation of the data degrades, precisions of the clustering and the like carried out later decrease, and an amount of calculation increases.

The first embodiment provides a method of selecting an appropriate number of dimensions for representing the positional relationship among the data based on the similarity among data. As a result, the data can be arranged on a vector space having the appropriate number of dimensions based on the similarity among the data.

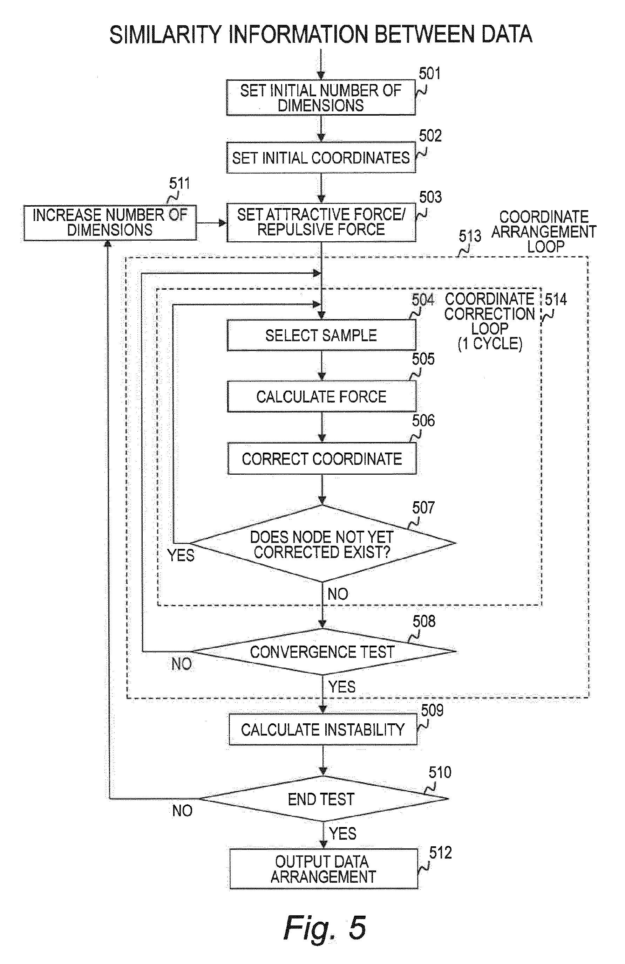

FIG. 5 is a flowchart of the processing by a data mapping unit 207 according to the first embodiment.

The data mapping unit 207 arranges the data in the space having the appropriate number of dimensions based on the similarity information between data 209.

First, in Initial number-of-dimensions setting step 501, an initial value of the dimension of the coordinates is set. The initial value of the dimension of the coordinates is set to as small a value as possible, but if it is known that the representation of the data apparently requires at least n dimensions (n is a positive number), n is set to the initial value. If the prior knowledge does not exist, the initial value for the dimension may be set to two.

Then, in Initial coordinate setting step 502, an initial arrangement of the coordinate $\{xi\}$ of each of the data is defined on the vector space having the number of dimensions set in Initial number-of-dimensions setting step 501. The initial arrangement of the coordinate $\{xi\}$ of the data may be set by using a random number, for example. Moreover, the data may be classified by means of a certain method, and nodes belonging to the same class may be arranged close to one another. Further, an arrangement defined by using another simple visualization algorithm may be the initial arrangement.

In Attractive force/repulsive force setting step 503, an attractive force and a repulsive force between data are defined. If a similarity between data $i$ and data $j$ is defined as $s(i,j)$, the attractive force $fa(i,j)$ between the data $i$ and the data $j$ is defined by using a predetermined threshold $h$ as Equation (11). $fa(i,j)=s(i,j)|xi-xj|^2/K$ if $s(i,j)\geq h$ holds true

$$fa(i,j)=0 \quad \text{if } s(i,j)<h \text{ holds true} \dots (11)$$

In other words, the acting attractive force is proportional to the similarity between the data $i$ and the data $j$ if the similarity is equal to or more than the predetermined threshold $h$. Moreover, the repulsive force is defined by Equation (1) as in the case illustrated in FIG. 4. The constants $C$ and $K$ are defined in advance.

Then, in a coordinate arrangement loop 513, the coordinate arrangement $\{xi\}$ of each of the data is successively corrected so as to reduce the energy E. As a result, a final arrangement of all the data is defined. The energy E is defined as in the case illustrated in FIG. 4. In other words, the force acting on the data $i$ is defined by Equation (4) as in the case illustrated in FIG. 4. Based on this fact, an energy of the entire system can be defined by Equation (6) as in the case illustrated in FIG. 4.

An algorithm iteratively corrects the coordinates $\{xi\}$ of the nodes so as to reduce the energy E. As a result, a final arrangement of all the data is defined.

A coordinate correction loop 514 is a cycle of successively selecting data, and correcting a coordinate of the data, and processing for one cycle is finished when the entire nodes are routed.

In Sample selection step 504, data $i$ is selected from the entire data. In Force calculation step 505, the sum $f(i)$ of the vectors of the force acting on the selected data $i$ is calculated. The sum $f(i)$ can be calculated by Equation (4).

In Coordinate correction step 506, the coordinate $xi$ is corrected in a direction of the force acting on the selected data $i$ by using Equation (9).

In Equation (9), $t$ is a parameter for adjusting a magnitude of the correction. The value of $t$ may initially be large for greatly correcting the coordinate, and may decrease for a final fine adjustment as the cycle repeats. For example, an initial value having an appropriate magnitude may be first set, and the value $t$ may be updated by using Equation (10) after the coordinate correction for one cycle is finished.

Then, in Step 507, whether data which has not been selected out of all the nodes exists or not is determined. As a result of the determination, if data which has not been selected out of all the data exists, the algorithm returns to Sample selection step 504, and selects a next sample. On this occasion, the force acting from the data $i$ to other data have changed as a result of the correction of the coordinate $xi$, and, in Force calculation step 505, the force vectors are calculated for the new coordinate arrangement by reflecting the correction of the coordinate. On the other hand, if all the data have been selected, the algorithm proceeds to Convergence determination step 508.

In Convergence determination step 508, a degree of convergence of the algorithm is determined, thereby determining whether the algorithm is finished or not. For example, a coordinate arrangement $\{yi\}$ of the data $i$ after one cycle of the coordinate correction for the last time and a coordinate arrangement $\{xi\}$ of the data $i$ after one cycle of the coordinate correction for this time are compared with each other, and if a correction amount is smaller than a predetermined threshold, the algorithm determines that the coordinates are sufficiently converged, and is finished. The correction amount may be calculated by using, for example, $\sum|xi-yi|$.

If it is determined that the coordinates have been converged, the algorithm proceeds to Instability calculation step 509.

A stability of the arrangement of data generated in Step 513 is calculated in order to appropriately select the number of dimensions according to the first embodiment. If the number of dimensions is insufficient, there is a problem in the positional relationship among the data, and it is assumed that a distortion is present in the force. The stability is evaluated by calculating a magnitude of the distortion. If the arrangement of data is stable, it is determined that the number of dimensions is sufficient. On the other hand, if the arrangement of data is not stable, in Number-of-dimensions addition step 511, the number of dimensions is incremented by one, and the coordinates of the data are arranged again in a space having the increased number of dimensions.

In Instability calculation step 509, the instability of the data arrangement generated in Coordinate arrangement step 513 is calculated by the following method. If the number of dimensions currently set is $N$, the data are arranged in an $N$-dimensional space. The instability is calculated by means of a degree of instability in the current arrangement if one dimension is virtually added to the dimension $N$. In other words, if the data is minutely fluctuated in a direction of the added dimension, and a force acts in a direction to pull back the fluctuation, the data arrangement is stable. On the other hand, if a force acts in the direction of the fluctuation, the data arrangement is unstable.

More specifically, the repulsive force between the data $i$ and the data $j$ is represented by Equation (1), and the attractive force is represented by Equation (11).

The attractive force acting on the data $i$ in the direction of the added dimension if the data $i$ is minutely fluctuated by $\delta$ in the direction of the added dimension is represented as Equation (12), and the repulsive force is represented by Equation (13).

$$fr(i,j) = -\delta C K^2 / (|xi-xj|^2 + \delta^2)$$

$$\approx -\delta C K^2 / |xi-xj|^2 \quad \ldots \quad (12)$$

$$fa(i,j) = \delta s(i,j)(|xi-xj|^2 + \delta^2)^{1/2} / K$$

$$\approx \delta s(i,j)|xi-xj| / K \text{ if } s(i,j) \geq h \text{ holds true}$$

$$fa(i,j) = 0 \quad \text{if } s(i,j) < h \text{ holds true } \ldots \quad (13)$$

On this occasion, if the values of *fr* and *fa* are positive, force acts in the direction to pull back the minute fluctuation in the direction of the added dimension, and the arrangement of the data is stable. On the other hand, if the values of *fr* and *fa* are negative, force acts in the direction to increase the minute fluctuation in the direction of the added dimension, and the arrangement of the data is unstable. Thus, a sum of force in the direction of the added dimension acting on the data *i* from all the data is represented by Equation (14).

$$u(i,\{xi\},K,C) = -\Sigma fr(i,j) - \Sigma fa(i,j) \quad \ldots \quad (14)$$

On this occasion, the sum is calculated for all the data *j* other than the data *i*. On this occasion, if $\delta$ is set to *K* by considering the constants *K* and *C* relating to the scaling of the coordinate, and scaling relating to the number *M* of data, a degree *U* of the instability is represented as Equation (15).

$$U = \Sigma u(i,\{xi\},K,C) / (KC^{1/3}M(M-1)) \quad \ldots \quad (15)$$

On this occasion, the sum is calculated for unstable data, namely, data satisfying a relationship, $u(i,\{xi\},K,C) > 0$. A denominator $M(M-1)$ is a term for normalizing by the number of sums caused by the number of samples, and $KC^{1/3}$ is a term for normalizing the coordinate scaling.

In Instability calculation step 509, *U* is calculated as an index representing the instability by the processing described before. Then, in Finish determination step 510, whether *U* is equal to or more than a predetermined threshold or not is determined. As a result of the determination, if *U* is equal to or more than the predetermined threshold, it is determined that the arrangement of data is unstable, and the algorithm proceeds to Number-of-dimensions addition step 511. On the other hand, if U is less than the predetermined threshold, it is determined that the data arrangement is stable, and the algorithm proceeds to Data arrangement output step 512 to output the coordinates of the data. If *U* decreases drastically compared with the previous cycle, it may be determined that the arrangement of data is stable.

In Final determination step 510, if it is determined that the arrangement of data is unstable, the algorithm returns to Number-of-dimensions addition step 511, and the number of dimensions is incremented by one. The coordinate in the added dimension of the data is set to O. In other words, if data *i* is arranged at a coordinate *xi=(xi1, xi2, ... , xiN)* in Coordinate arrangement step 513, the initial coordinate of the data *i* in the *N*+1-dimensional space is set to *xi=(xi1, xi2, ... , xiN, 0)*. Moreover, in Attractive force/repulsive force setting step 503, attractive force and repulsive force is set in the *N*+1-dimensional space. Then, in Coordinate arrangement step 513, a coordinate arrangement is determined in the *N*+1-dimensional space.

The above-described processing can arrange the data in the space having the appropriate number of dimensions. The number of dimensions is set to N, and the coordinate of the data *i* is set to *xi=(xi1, xi2, ... , xiN)*.

The example of using Force Directed Algorithm, but Multilevel Force Directed Algorithm has been described above, which is improved Force Directed Algorithm, may be applied to the method described above.

The clustering unit 208 clusters data arranged in the *N*-dimensional space. The data are represented as points in the *N*-dimensional space, and hence the K-means method or the EM method can be used for the clustering.

For example, the K-means method first defines the number *K* of clusters. Then, each of the pieces of data is randomly assigned to any of the clusters. Then, an average vector of data belonging to a cluster is acquired for each of the clusters. Then, each of the pieces of data is assigned to a cluster corresponding to the closest average vector. If a change does not arise as a result of the reassignment, the clustering is finished. On the other hand, if a change occurs as a result of the reassignment, the process returns to the processing of acquiring the average vector of data belonging to a cluster for each of the clusters, and the processing is repeated. As a result of the processing, the data can be classified into *K* clusters.

As described above, the data clustering apparatus according to the first embodiment of this invention can arrange data based on the similarity between data in a vector space having an appropriate number of dimensions. For example, if the similarity between people or documents is given, this embodiment is suitably used for classification into similar people or similar documents.

Moreover, the data is arranged in the vector space having an appropriate number of dimensions, and the related-art clustering method can thus be used to appropriately classify the instances. In other words, there is a related-art classification method of clustering points arranged at positions close to one another in the *N*-dimensional space, but the points may not be appropriately classified by means of the related-art clustering method depending on the number of dimensions of the space in which pieces of data are arranged. Therefore, according to the first embodiment, an appropriate number of dimensions of the vector space is determined based on the similarity between data, and the data are arranged in the space having the number of dimensions. The related-art clustering method can thus be applied.

Moreover, the instability of the arrangement of data is calculated, thereby determining an appropriate number of dimensions. Therefore, the appropriate number of dimensions can be determined automatically without necessity of a prior knowledge on the data.

[Second embodiment]

According to a second embodiment of this invention, a description is given of an example of a medical data analysis system for selecting a healthcare guidance subject person, proposing a healthcare guidance method, and estimating a healthcare guidance effect in order to prevent a disease onset and an increase in severity of a disease based on medical data (such as healthcare cost information, health checkup information, and medical inquiry information).

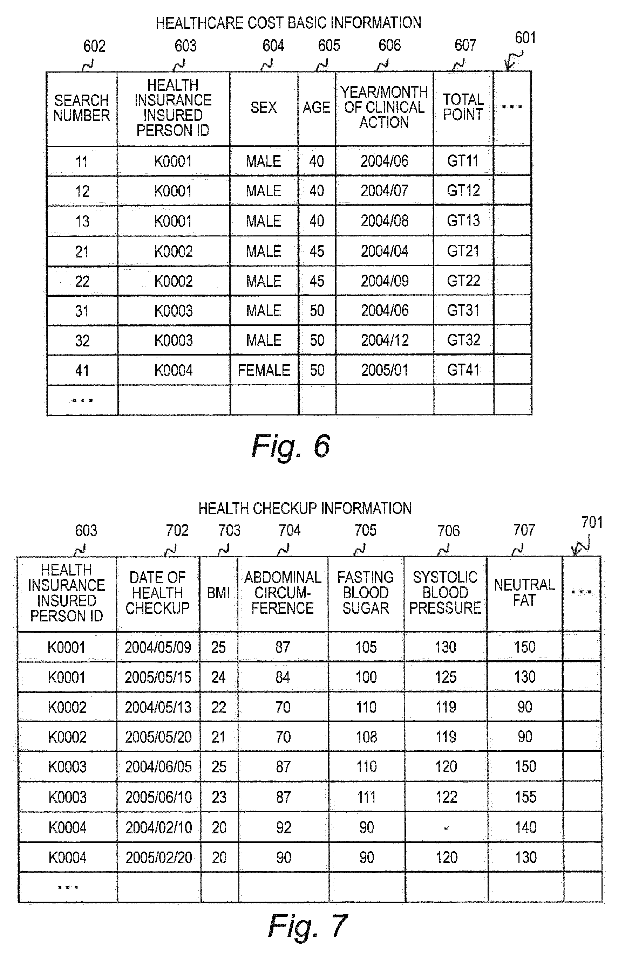

The healthcare cost information is information recording names of injury and illness when a health insurance insured person visits a medical institute for consultation, prescribed medicines, applied clinical actions, and medical costs (points), and, referring to FIG. 6, a description is given of an example thereof. The prescribed medicines and the applied clinical actions are generally referred to as medical action.

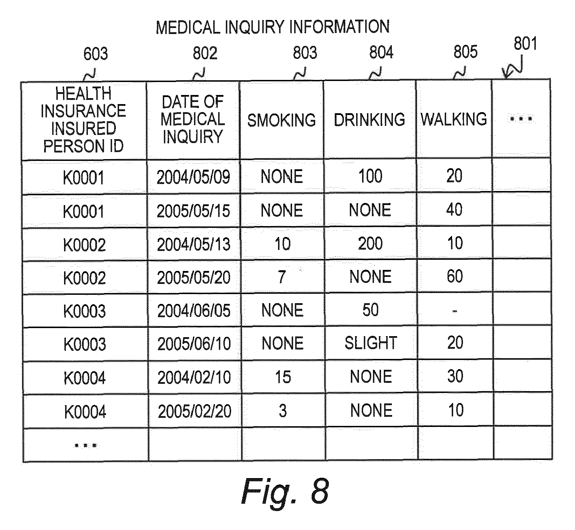

Moreover, the health checkup information is information on a result of a test received by an insured person to the health insurance at a health checkup facility, and, referring to FIG. 7, a description is later given of an example thereof. The medical inquiry information is information on a result of a medical inquiry (such as the life style, health history, and subjective symptoms) received at a health checkup facility by an insured person of the health insurance at a health checkup facility, and, referring to FIG. 8, a description is later given of an example thereof

Insured persons of the health insurance in a similar state are clustered based on medical data according to the second embodiment. The cluster represents states of a disease (absence/presence of a disease onset, the degree of severity, the test value, and the like). A model based on state transitions between the clusters is generated, a tendency of all the insured persons is analyzed from statistics of insured persons in a cluster based on the generated model, a future state is estimated, and a medical cost is estimated according to the second embodiment.

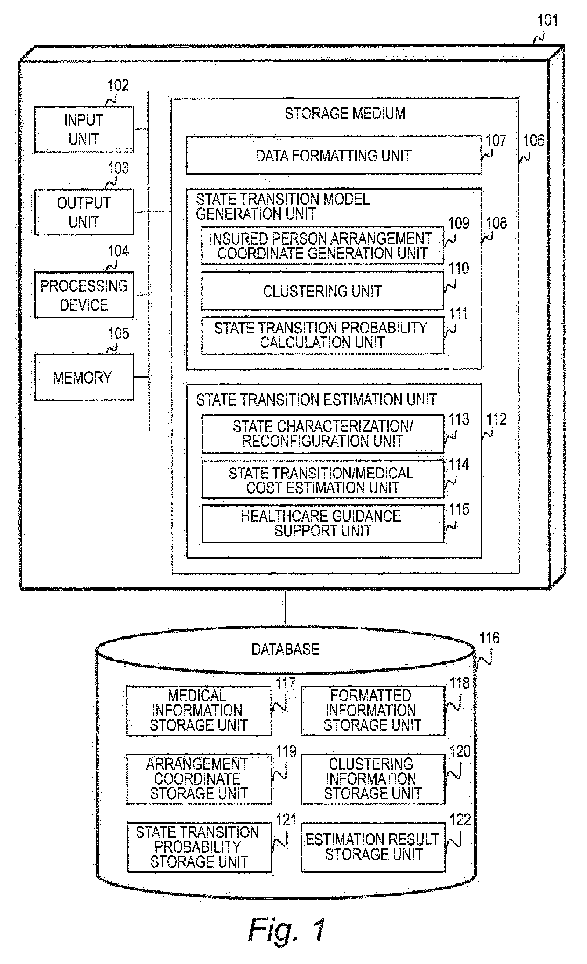

FIG. 1 is a block diagram illustrating a configuration of the medical data analysis system according to the second embodiment.

The medical data analysis system includes a medical data analysis apparatus 101 and a database 116.

The medical data analysis apparatus 101 according to this embodiment includes an input unit 102, an output unit 103, a processing device 104, a memory 105, and a storage medium 106.

The input unit 102 is a human interface such as a mouse and a keyboard, and receives an input to the medical data analysis apparatus 101. The output unit 103 includes a display and a printer for outputting an arithmetic operation result by the medical data analysis system. The storage medium 106 is a storage apparatus for storing various programs for realizing medical data analysis processing by the medical data analysis system, an execution result of the medical data analysis processing, and the like, and is, for example, a non-volatile storage medium (such as a magnetic disk drive and non-volatile memory). The programs stored in the storage medium 106 are extended on the memory 105. The processing device 104 is an arithmetic operation apparatus for executing a program loaded on the memory 105, and is, for example, a CPU, a GPU, or the like. Processing and arithmetic operation described later are carried out by the processing device 104.

The medical data analysis system according to this embodiment may be a computer system constituted by a single computer, or a computer system constituted by a server and client terminals.

The medical data analysis system is a computer system constituted on a single computer, or a plurality of logically or physically constituted computers, and may operate as independent threads on the same computer, or may operate on virtual computers constructed on a plurality of physical computer resources.

The program executed by the processor is provided for respective servers by means of a removable medium (such as a CD-ROM and a flash memory) or a network, and is stored in a non-volatile storage apparatus which is a non-transitory storage medium. Therefore, the computer system is preferred to include an interface for reading the removable medium.

First, a description is given of medical data used in the second embodiment.

A medical information storage unit 117 stores the medical data input to the input unit 102. The medical data includes the healthcare cost information, the health checkup information, and the medical inquiry information. The healthcare cost information includes healthcare cost basic information, injury and illness name information, clinical action information, medicine information, injury and illness name classification information, clinical action classification information, and medicine classification information.

A description is now given of the healthcare cost information.

FIG. 6 is an explanatory diagram illustrating healthcare cost basic information 601.

The healthcare cost basic information 601 is information holding relationships between a healthcare cost and an insured person. The healthcare cost basic information 601 includes search numbers 602, insured person IDs 603, sex 604, ages 605, months and years of clinical action 606, and total points 607.

The search number 602 is an identifier for uniquely identifying a healthcare cost record. The insured person ID 603 is an identifier for uniquely identifying an insured person of the health insurance. The sex 604 is information representing a gender of the insured person. The age 605 is information representing an age of the insured person.

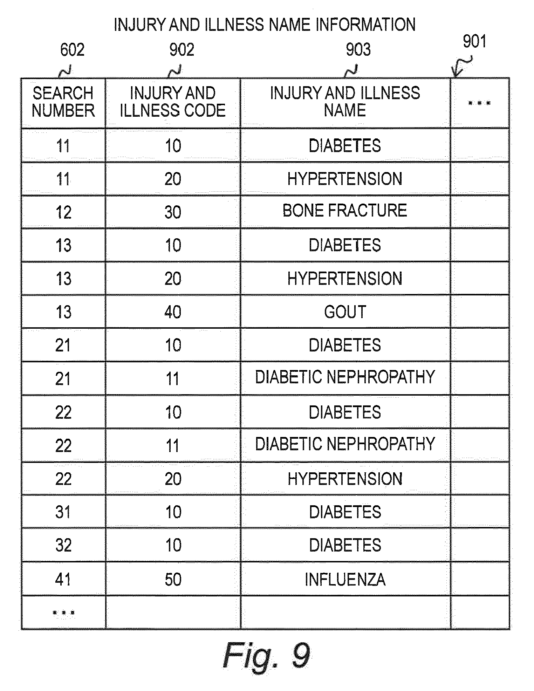

The month and year of clinical action 606 is a month and a year when the insured person visits a medical institute. The total point 607 is information representing a total point of one healthcare cost. It should be noted that a medical cost (in yen) is calculated by multiplying the total point by "10". If a plurality of injury and illness names are registered to one search number in injury and illness name information 901 illustrated in FIG. 9, a total point of the medical actions for the plurality of injuries/illnesses is registered to the total point 607.

FIG. 9 is an explanatory diagram illustrating the injury and illness name information 901.

The injury and illness name information 901 includes search numbers 602, injury and illness codes 902, and injury and illness names 903.

The search number 602 is an identifier for uniquely identifying the healthcare cost record, and uses the same number as the search number (FIG. 6) of the healthcare cost basic information 601. The injury and illness code 902 is an injury and illness code described in the healthcare cost record. The injury and illness name 902 is an injury and illness name corresponding to the injury and illness code.

It should be noted that a plurality of injury and illness names can be described in one healthcare cost record. For example, the injury and illness names 903 of entries having "11" in the search number 602 are "diabetes" and "hypertension" in the injury and illness name information 901 illustrated in FIG. 9, and the injury and illness names, which are diabetes and hypertension, are described in the healthcare cost records having "11" in the search number.

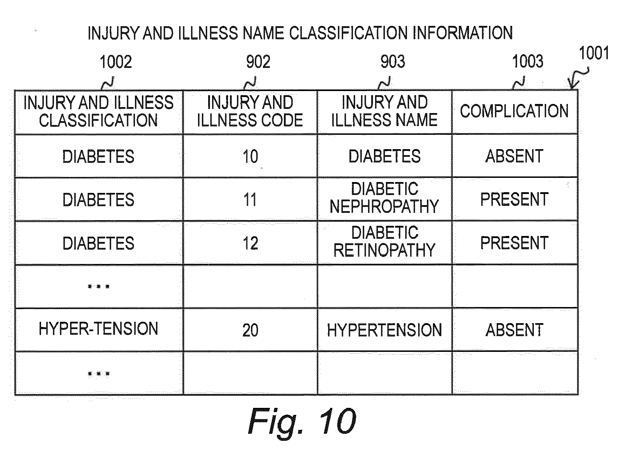

FIG. 10 is an explanatory diagram illustrating the injury and illness name classification information.

Injury and illness name classification information 1001 is information for associating an injury and illness classification and an injury and illness name belonging to the injury and illness classification with each other, and includes an injury and illness classification 1002, an injury and illness code 902, an injury and illness name 903, and a complication 1003.

The injury and illness classification 1002 is the classification to which an injury and illness in question belongs. The injury and illness code 902 is an injury and illness code described in healthcare cost record, and uses the same numbers as used in the injury and illness code 902 of the injury and illness name information 901 illustrated in FIG. 9. The injury and illness name 903 is the name of an injury and illness corresponding to this injury and illness code, and uses the same names as used in the injury and illness name 903 of the injury and illness name information 901 illustrated in FIG. 9. The complication absence/presence 1003 indicates whether this injury and illness is the name of a complication

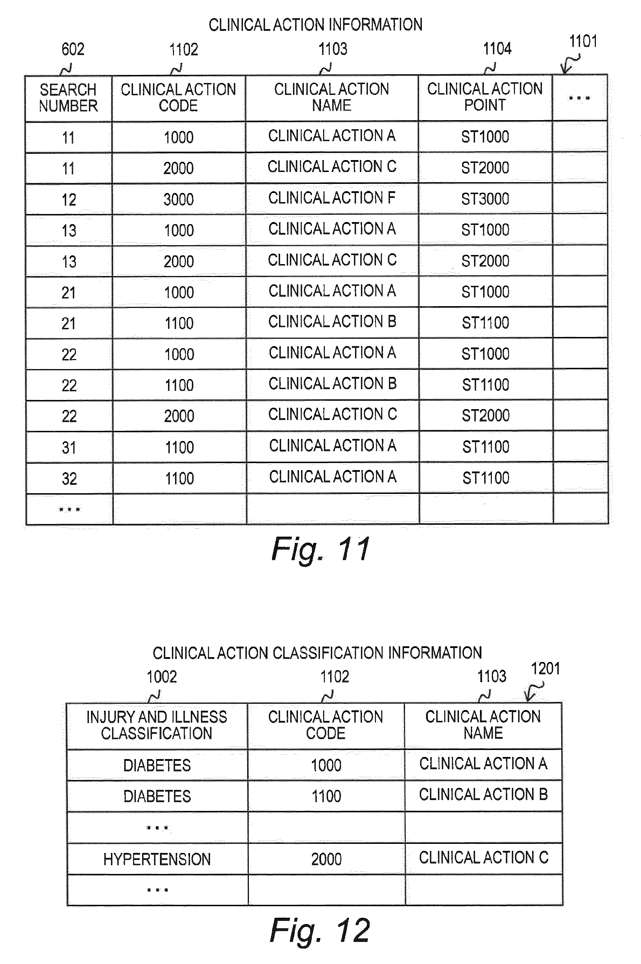

FIG. 11 is an explanatory diagram illustrating the clinical action information.

Clinical action information 1101 includes search numbers 602, clinical action codes 1102, clinical action names 1103, and clinical action points 1104.

The search number 602 is an identifier for uniquely identifying a healthcare cost record, and uses the same number as the search number (FIG. 6) of the healthcare cost basic information 601. The clinical action code 1102 is an identifier for identifying a clinical action described in the healthcare cost record. The clinical action name 1103 is a name of the clinical action described in the healthcare cost record. The clinical action point 1104 is a point relating to the clinical action.

In FIG. 11, for example, the clinical action names 1103 of "clinical action A" and "clinical action C" are described in healthcare cost records having "11" in the search number 602.

FIG. 12 is an explanatory diagram illustrating the clinical action classification information.

Clinical action classification information 1201 includes injury and illness classifications 1002, clinical action codes 1102, and clinical action names 1103.

The injury and illness classification 1002 uses the same classification as the injury and illness classification 1002 (FIG. 10) of the injury and illness name classification information 1001. The clinical action code 1102 is a clinical action code for identifying a clinical action practiced for the injury and illness in the injury and illness classification 1002, and uses the same code as the clinical action code 1102 (FIG. 11) of the clinical action information 1101. The clinical action name 1103 is a name of the clinical action corresponding to the clinical action code, and uses the same code as the clinical action name 1103 (FIG. 11) of the clinical action information 1101.

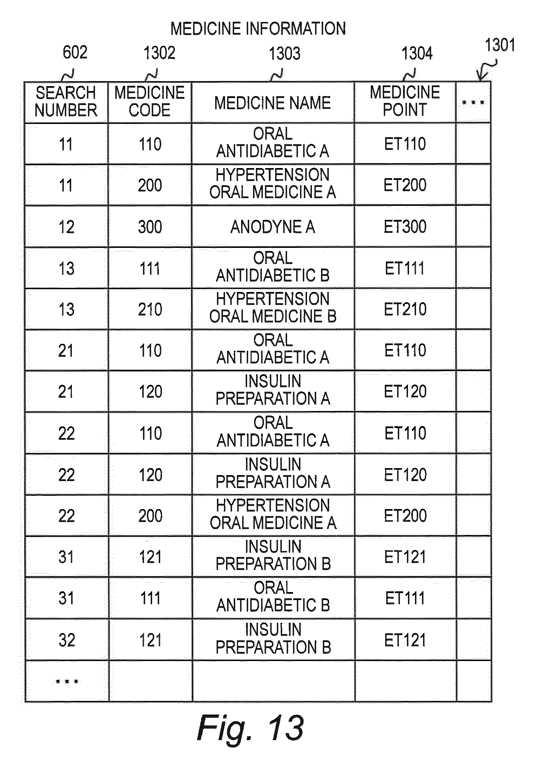

FIG. 13 is an explanatory diagram illustrating the medicine information.

Medicine information 1301 includes search numbers 602, medicine codes 1302, medicine names 1303, and medicine points 1304.

The search number 602 is an identifier for uniquely identifying a healthcare cost, and uses the same number as the search number 602 (FIG. 6) of the healthcare cost basic information 601. The medicine code 1302 is a medicine code for identifying the medicine described in the healthcare cost record. The medicine name 1303 is a name of a medicine described in the healthcare cost record. The medicine point 1304 is an insurance point of the medicine.

In FIG. 13, for example, the medicine names of "oral antidiabetic A" and "hypertension oral medicine A" are described in the healthcare cost records having "11" in the search number 602.

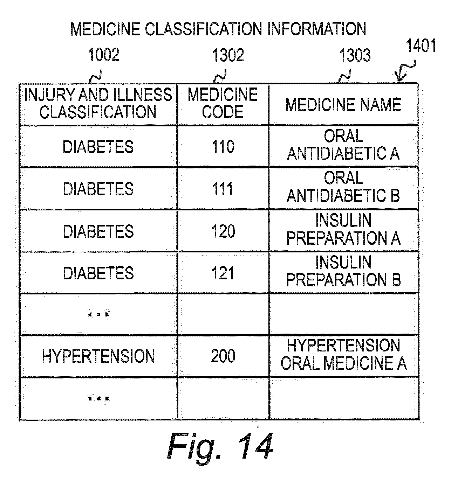

FIG. 14 is an explanatory diagram illustrating the medicine classification information.

Medicine classification information 1401 includes injury and illness classifications 1002, medicine codes 1302, and medicine names 1303.

The injury and illness classification 1002 uses the same classification as the injury and illness classification 1002 (FIG. 10) of the injury and illness name classification information 1001. The medicine code 1302 is a medicine code for identifying a medicine prescribed in the classification registered to the injury and illness classification 1002, and uses the same code as the medicine code 1302 (FIG. 13) of the medicine information 1301. The medicine name 1303 is a name of a medicine corresponding to the medicine code, and uses the same name as the medicine name 1301 (FIG. 13) of the medicine information 1303.

It should be noted that the clinical action information 1101 illustrated in FIG. 11 and the medicine information illustrated in FIG. 13 are generally referred to as medical action information. It should be noted that the clinical action classification information 1201 illustrated in FIG. 12 and the medicine classification information illustrated in FIG. 14 are generally referred to as medical action classification information.

A description is now given of the health checkup information.

FIG. 7 is an explanatory diagram illustrating the health checkup information.

Health checkup information 701 is information for managing health checkup information on a plurality of insured persons for a plurality of years, and includes insured person IDs 603, health checkup dates 702, and various test values (such as BMIs 703, abdominal circumferences 704, fasting blood sugars 705, systolic blood pressures 706, and neutral fats 707) in the health checkup.

The insured person ID 603 is an identifier of an insured person to the health insurance who has had the health checkup, and uses the same identifier as the insured person ID 603 (FIG. 6) of the healthcare cost basic information 601. The health checkup date 702 is the date of the health checkup. The BMI 703 to the neutral fat 707 are results of tests in the health checkup.

For example, if an insured person has not had a specific test, data in the health checkup information may be absent. For example, in FIG. 7, the items of a test received in 2004 of an insured person having an insured person ID "K0004" lack data for the systolic blood pressure 706.

A description is now given of the medical inquiry information.

FIG. 8 is an explanatory diagram illustrating the medical inquiry information.

Medical inquiry information 801 is information for managing medical inquiry information on a plurality of insured persons for a plurality of years, and includes insured person IDs 603, medical inquiry dates 802, and answers of the medical inquiry (such as smoking 803, drinking 804, and walking 805. The medical inquiry may include a life style, health history, a constitution such as allergy, and subjective symptoms.

The insured person ID 603 is an identifier of an insured person of the health insurance who has had the medical inquiry, and uses the same identifier as the insured person ID 603 (FIG. 6) of the healthcare cost basic information 601. The medical inquiry date 802 is the date of the medical inquiry. The smoking 803 to the walking 805 are results of the medical inquiry. The smoking 803 represents an average number of cigarettes smoked per day if the insured person has the smoking habit, and is "none" if the insured person does not smoke. The drinking 804 represents an average amount (unit: ml) of alcoholic beverages drunk per day if the insured person has the drinking habit, and is "none" if the insured person does not have the drinking habit. The walking 805 is an average period (unit: minute) of walking per day.

Detailed information such as the number of steps in the walking, the amount of drinking, and the number of smoked cigarettes may not be acquired from the medical inquiry. Not a specific amount of drinking, but a corresponding frequency out of frequencies classified in advance in a questionnaire may be responded. For example, if information only on absence/presence of habits of smoking and drinking are acquired, the frequency of drinking may be divided into a certain number of degrees (such as (1) none, (2) once to twice per week, (3) three times or more per week), and the frequency may be responded. In this case, the value in the medical inquiry information is the number without a quantitative meaning.

If an answer to a specific item is not received, data on the medical inquiry may be absent. For example, in FIG. 8, the items of a medical inquiry received in 2004 by an insured person having an insured person ID "K0003" lack data for the walking 805.

A description is now given of processing for a data formatting unit 107. The data formatting unit 107 sums/unifies and formats in a tabular form the healthcare cost information, the health checkup information, and the medical inquiry information for each of the insured persons and each period from the medical data stored in the medical information storage unit 117. In the following, a description is given while assuming one period is one year, but the one period may be another period such as half a year, two years, and three years.

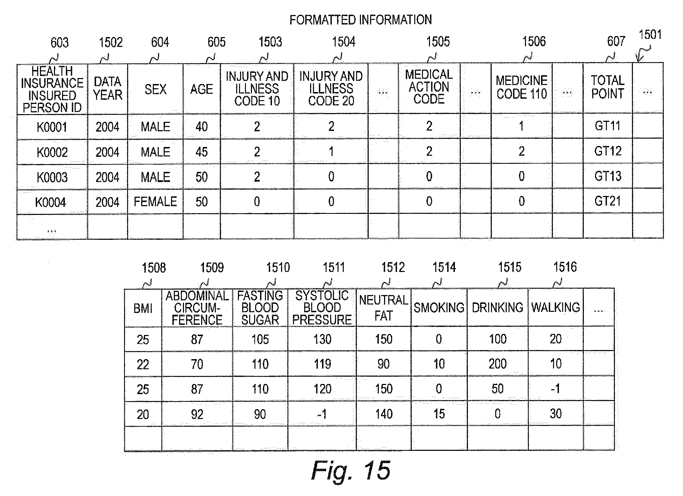

FIG. 15 is an explanatory diagram illustrating an example of formatted information 1501. Referring to FIG. 15, a description is given of the processing by the data formatting unit 107.

The formatted information 1501 includes healthcare cost formatted information acquired by formatting the healthcare cost information in the year of 2004. Each row of the formatted information 1501 represents data summed for one insured person ID for one year.

An insured person ID 603, a sex 604, an age 605, and a total point 607 are the same as the insured person ID 603, the sex 604, the age 605, and the total point 607 (FIG. 6) of the healthcare cost basic information 601. A data year 1502 is a year of source data from which the formatted information is generated.

The injury and illness code 10 (1503) is the number of healthcare cost records having "10" in the injury and illness code out of the healthcare cost records for the insured person ID. The injury and illness code 20 (1504) is similarly the number of healthcare cost records having "20" in the injury and illness code out of the healthcare cost records for the insured person ID. The clinical action code 1000 (1505) is the number of healthcare cost records for which a clinical action having "1000" in the clinical action code has been performed out of the healthcare cost records for the insured person ID. The medicine code 110 (1506) is the number of healthcare cost records for which a medicine having the medicine code of "110" has been prescribed out of the healthcare cost records for the insured person ID.

A specific description is now given of the processing by the data formatting unit 107 for a case where the data in the year of 2004 are formatted.

First, one insured person ID is selected. The data formatting unit 107 acquires search numbers of healthcare cost records for the insured person ID having "2004" in the month and year of clinical action from the healthcare cost basic information 601. Then, the data formatting unit 107 refers to the injury and illness name information 901, and counts, for each injury and illness code, the number of healthcare cost records having the injury and illness code described thereon. As a result, the number of healthcare cost records for each of the injury and illness codes is acquired. Similarly, the data formatting unit 107 refers to the clinical action information 1101, counts the number of healthcare cost records for each of the clinical action codes, refers to the medicine information 1301, and counts the number of healthcare cost records for each of the medicine codes. As a result, a data row for the year of 2004 is generated for the selected insured person ID. This processing is carried out for all combinations of each of insured person IDs and each of the years subject to the analysis.

For example, search numbers "11", "12", and "13" can be acquired from the healthcare cost basic information 601 for the data of the insured person ID "K0001" in the first row in 2004 in the formatted information 1501 illustrated in FIG. 15. Referring to the injury and illness name information 901, the number of healthcare cost records having "10" in the injury and illness code is two corresponding to the search numbers "11" and "13" out of the three healthcare cost records. Thus, 2 is registered to the column of the injury and illness code 10 in the first row of the formatted information 1501.

The formatted information 1501 illustrated in FIG. 15 includes health checkup formatted information formatted from the health checkup information. Each row includes sums of data corresponding to a single insured person ID.

A value of each of the items is a value of the health checkup data of an insured person and a year respectively corresponding to the insured person ID 603 and the data year 1502. The health checkup data can be acquired from the health checkup information 701. If the health checkup information 701 includes a plurality of pieces of health checkup data corresponding to the same insured person ID and the same year, data on any one of dates of health checkup or an average of health checkup results of the plurality of times for the year may be used. If the data on one health checkup date is used, it is preferred to use data on a simultaneous health checkup day practiced at approximately the same time of every year. Moreover, data small in amount of deficiency may be selected. A numerical value defined in advance for representing the deficiency is used for the deficient data. In the example illustrated in FIG. 15, -1 is used. It should be noted that all values for an insured person who does not have the health checkup information are treated as the values of the deficient data.

The formatted information 1501 illustrated in FIG. 15 includes medical inquiry formatted information formatted from the medical inquiry information. Each row includes sums of data corresponding to a single insured person ID.

A value of each of the items is a value of the medical inquiry data of an insured person and a year respectively corresponding to the insured person ID 603 and the data year 1502. The medical inquiry data can be acquired from the medical inquiry information 801. If the medical inquiry information 801 includes a plurality of pieces of medical inquiry data corresponding to the same insured person ID and the same year, data on any one of the dates of medical inquiry or an average of medical inquiry results of the plurality of times for the year may be used. If the data on one health checkup date is used, it is preferred to use data on a simultaneous health checkup day practiced every year at approximately the same time of every year. Alternatively, data small in amount of deficiency may be selected. A numerical value defined in advance for representing the deficiency is used for the deficient data. In the example illustrated in FIG. 15, -1 is used. It should be noted that all values for an insured person who does not have the medical inquiry information are treated as the values of the deficient data.

As a result of the processing, healthcare cost formatted information, health checkup formatted information, and medical inquiry formatted information can be generated. The data only for the year of 2004 is illustrated in FIG. 15, but pieces of formatted data for other years are also generated.

On this occasion, when the healthcare cost formatted information is generated, similar items may be summarized, thereby unifying the plurality of items. For example, if functions of the oral antidiabetic A and functions of the oral antidiabetic B out of the items of the medicines are similar, the oral medicines A and B may be summarized, and may be treated as one item. On this occasion, a sum of the number of prescriptions of the oral antidiabetic A and the number of prescriptions of the oral antidiabetic B in the same year is used as a value of the item newly summarized. Criteria for determining whether items are similar or not are described below. Clinical action names belonging to the same injury and illness classification in the clinical action classification information 1201 are considered as similar items. Moreover, medicine names belonging to the same injury and illness classification in the medicine classification information 1401 are considered as similar items. Moreover, similar item information is manually generated in advance.

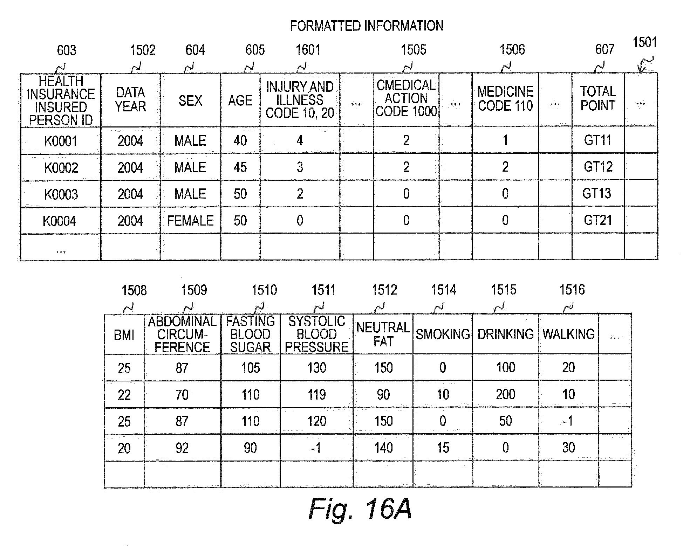

FIG. 16A is an explanatory diagram illustrating an example of the formatted information 1501 acquired by unifying the injury and illness code 10 and the injury and illness code 20 of the healthcare cost formatted information. The value of an injury and illness code 1601 is a sum of the value of the injury and illness code 1503 and the value of the injury and illness code 1504 in FIG. 15, and is a sum of the number of healthcare cost records having "10" in the injury and illness code and the number of healthcare cost records having "20" in the injury and illness code.

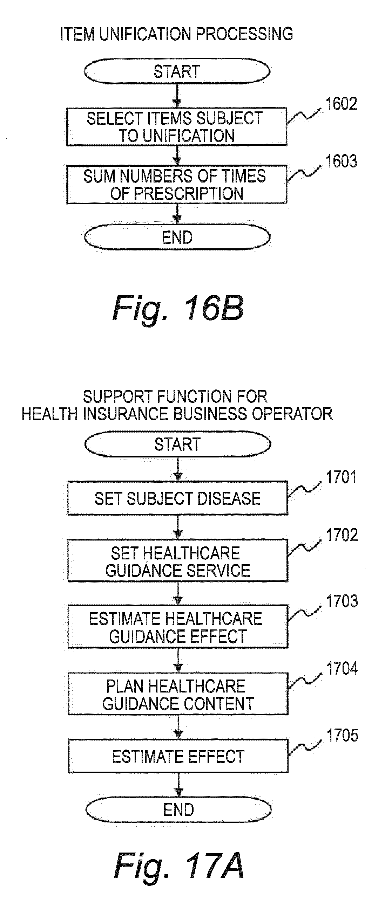

FIG. 16B is a flowchart of item unification processing.

First, in Unification subject item selection step 1602, items subject to the unification are selected out of the items in the healthcare cost basic information 601. A description is given of three examples of selecting the items. A first example is a method of considering items belonging to the same injury and illness class as the unification subjects if the clinical action and the medicines in the clinical action classification information 1201 and the medicine classification information 1401 are classified. A second example is a method of unifying items belonging to the same class by using a criterion such as the 10th Revision of International Classification of Diseases (IDC 10). A third example is a method of clustering the items by using the same method as that by the clustering unit 110 described later, and unifying items belonging to the same cluster.

Then, in Number of times of prescription summing step 1603, values of the items to be unified are summed.

The generated healthcare cost formatted information, health checkup formatted information, and medical inquiry formatted information illustrated in FIG. 15 and 16A are stored in a formatted information storage unit 118 of the database 116. The formatted information 1501 is numerical data in a tabular form.

The value in the healthcare cost formatted information is acquired by summing the number of the healthcare cost records, namely the number of prescriptions, but the value may be information representing whether the prescriptions exist or not. In other words, a case where the number of prescriptions is equal to or more than 1 (prescription exists) is summarized as 1, and a case where the number of prescriptions is 0 (no prescription) is set to 0, resulting in a binary representation. Moreover, the number of prescriptions may be considered to represent the severity, and the value of the healthcare cost formatted information may be a value representing a level as a result of classification of the number of prescription. For example, a case where the number of prescriptions is 0 is set to 0, a case where the number of prescriptions is 1 to 4 is set to 1, and a case where the number of prescriptions is equal to or more than 5 is set to 2, resulting in a representation as the three stages.

In the above described example, the healthcare cost formatted information, the health checkup information, and the medical inquiry information are summarized for the period of one year. However, the period may be set to a different period of two years, three years, or the like. In the following, a description is given of a case where the period for the summarizing is one year.

A description is now given of a state transition model generation unit 108. The state transition model generation unit 108 includes an insured person arrangement coordinate generation unit 109, a clustering unit 110, and a state transition probability calculation unit 111.

The insured person arrangement coordinate generation unit 109 uses the formatted information to calculate the similarity between insured persons, and arranges the insured persons in the space having the appropriate number of dimensions based on the similarities. The insured person arrangement coordinate generation unit 109 stores the coordinates in the space where the insured persons are arranged in the arrangement coordinate storage unit 119. The clustering unit 110 clusters (classifies) the insured persons based on the arrangement of the insured persons in the space. The method according to the first embodiment can be used for the arrangement of the insured persons based on the similarity in the space and for the clustering. The clustering unit 110 stores a result of the clustering of the insured persons in a clustering information storage unit 120.

A description is now given of the method of calculating the similarity between the insured persons of the health insurance. It should be noted that the same insured person in different years is treated as different people. In other words, if medical data for ten thousand people for three years exists, the clustering is carried out for thirty thousand people. If a dissimilarity in an item $i$ between two insured persons is represented as $d(i)$, a dissimilarity between the two insured persons is calculated as $D=\sum d(i)$, which is a sum of the dissimilarities of an item used for calculating the dissimilarities. On this occasion, the sum is calculated for all the items $i$ for the same year used for calculating the dissimilarities. If a specific disease (such as diabetes) is focused on, the medical action corresponding to the disease in the healthcare cost information can be selected as the item used for calculating the dissimilarity.

A description is now given of a calculation method for the dissimilarity $d(i)$ for the item $i$. If the item $i$ is an item which does not have a quantitative meaning such as a reply number in a medical inquiry, $d(i)$ is set to 0 if replies of two insured persons are the same, and $d(i)$ is set to $v$ if the replies of the two insured persons are not the same. It should be noted that a predetermined value is used as $v$.

A description is now given of a case where the item $i$ has a quantitative meaning such as the number of prescriptions or a test value. If the item $i$ is an item having a quantitative meaning, a cumulative probability $F(t)=P(X\leq t)$ of the value is acquired. $F(t)$ is a ratio of the number of insured persons equal to or less than $t$ in the value of the item to the number of all the insured persons, and the maximum value of $F(t)$ is 1. Then, if the values of the item of the two insured persons are respectively $t1$ and $t2$, $d(i)$ is set to $|F(t1)-F(t2)|$.

If any one of the two insured persons is deficit in the value in the item $i$, $d(i)$ is set to $w$. It should be noted that a predetermined value is used as $w$.

As a result, the dissimilarity $D=\sum d(i)$ can be calculated. The similarity can be calculated as, for example, $-D+\max\{d(i)\}$. As described above, the insured persons can be classified by clustering the insured persons.

By the way, as illustrated in FIG. 16A, the formatted information where similar diseases are unified may be used for the calculation of the dissimilarity. If the formatted information where similar diseases are unified is used, clinical actions having similar effects are summarized and counted, and a dissimilarity close to an actual situation can thus be acquired. The clinical action classification information (FIG. 12) and the medicine classification information (FIG. 14) may be used for the unification of the clinical actions. Moreover, another unification method described below may be used.

The another unification method calculates the similarity between items, clusters the items by using the method according to the first embodiment, and unifies the clustered items.

A description is now given of a method of calculating the similarity between items. Values of two items arranged in a sequence of the insured person ID are respectively set to $x1=(x11, x12, ..., x1n)$ and $x2=(x21, x22, ... , x2n)$. A correlation coefficient between the vectors $x1$ and $x2$ are represented as $r(x1,x2)$. By the way, $x1$ and $x2$ include deficit values, and hence elements which are deficit in any one of $x1$ and $x2$ are removed. For example, if $x1i$ is deficit, $x2i$ is also removed. In this way, the vectors acquired by removing the deficit dimensions from $x1$ and $x2$ are respectively represented as $v1=(v11, v12, ... , v1m)$ and $v2=(v21, v22, ... , v2m)$.

Even if $v1$ and $v2$ have the same degree of dependencies, a deviation is generated in the correlation value $r(v1,v2)$ depending on a difference in nature of values between $v1$ and $v2$. Thus, first, if vectors $w1$ and $w2$ are acquired by independently and randomly rearranging the elements of $v1$ and $v2$, it is assumed that a degree of dependency does not exist between the vectors $w1$ and w2. Based on this fact, $|r(v1,v2)|-|r(w1,w2)|$ is calculated. It can be determined that, if a relationship $|r(v1,v2)|<|r(w1,w2)|$ holds true, the dependency does not exist. Therefore, the dependency is considered to be 0 in this case, and $|r(v1,v2)|-|r(w1,w2)|$ is considered as the dependency in the other cases. As a result, the similarity compared with the random case (case of no dependency) can be calculated.

As described above, the insured persons can be classified by using the unified items, thereby clustering the insured persons.

The insured persons are classified into $K$ clusters, $C1$, $C2$, ... , $CK$. Each of the clusters represents a state of a disease, and the insured person is classified to any of the clusters according to the second embodiment. It should be noted that pieces of data of the same person in different years are treated as data of different people as described before.

The state transition probability calculation unit 111 calculates a transition probability between clusters. A specific description is given of a method of calculating a transition probability from a cluster $Ci$ to a cluster $Cj$. First, the number of insured persons having data for a next year out of insured persons belonging to the cluster $Ci$ is set to $M1$. The number of insured persons in the state $Cj$ in the next year out of the insured persons is set to $M2$. The transition probability from the cluster *Ci* to the cluster *Cj* is represented as *M2/M1*. The state transition probability calculation unit 111 stores the values of the transition probability of all combinations of the clusters *Ci* and *Cj* in a state transition probability storage unit 121.

A description is now given of a state transition estimation part 112. The state transition estimation unit 112 includes a state characterization/reconfiguration unit 113, a state transition/medical cost estimation unit 114, and a healthcare guidance support unit 115.

The state characterization/reconfiguration unit 113 provides a state with meaning (characterizes the state), thereby reconfiguring the model. The insured persons are classified by means of the clustering, thereby configuring clusters in the model generated by the state transition model generation unit 108. Transition between states of a disease is modeled by acquiring the state transition probability between the configured clusters. However, the cluster is a set of insured persons, and the cluster needs to be characterized. How a disease is onset or how a severity of a disease increases can be intuitively recognized by characterizing clusters, and displaying states of diseases represented by the clusters on the output unit.

First, a description is given of a method of characterizing a cluster. A cluster is characterized by insured persons belonging to the cluster.

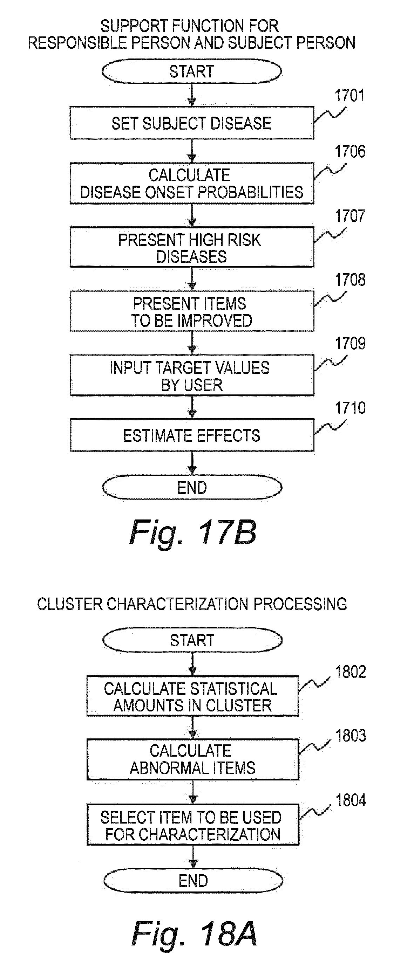

FIG. 18A is a flowchart of cluster characterization processing.

First, in Intra-cluster statistical amount calculation step 1802, a statistical amount of values of each of the items of insured persons belonging to a cluster is calculated, and a state of the cluster is represented by the calculated statistical amount. For example, the state can be represented by an average of the values of each of the items of the insured persons belonging to the cluster. For example, a blood sugar level of the cluster is an average of the blood sugar levels of the insured persons belonging to the cluster or the like.

Then, in Abnormal item calculation step 1803, a deviation rate of each of the insured persons from an average value is calculated for each of the items of the cluster. The deviation rate is calculated by using a method described below. For example, a value for a certain item of the cluster is set to *k*. A rate of people having a value equal to or less than *k* in this item out of all the insured persons is Y, and a rate of people having a value less than k in this item is *X*. On this occasion, in the case of $Z=(X+Y)/2$, it is considered that the values of the cluster relating to this item are closer to an average value as the Z is closer to 0.5. Conversely, as Z becomes more than or less than 0.5, the deviation rate increases. Then, the deviation rate is calculated as |Z-0.5|. This means that if values of a certain item in a cluster are greatly different from the average of all the insured persons, or deviate from a normal value, the deviation rate is large. An item larger in the deviation rate is more efficient for characterizing the cluster.

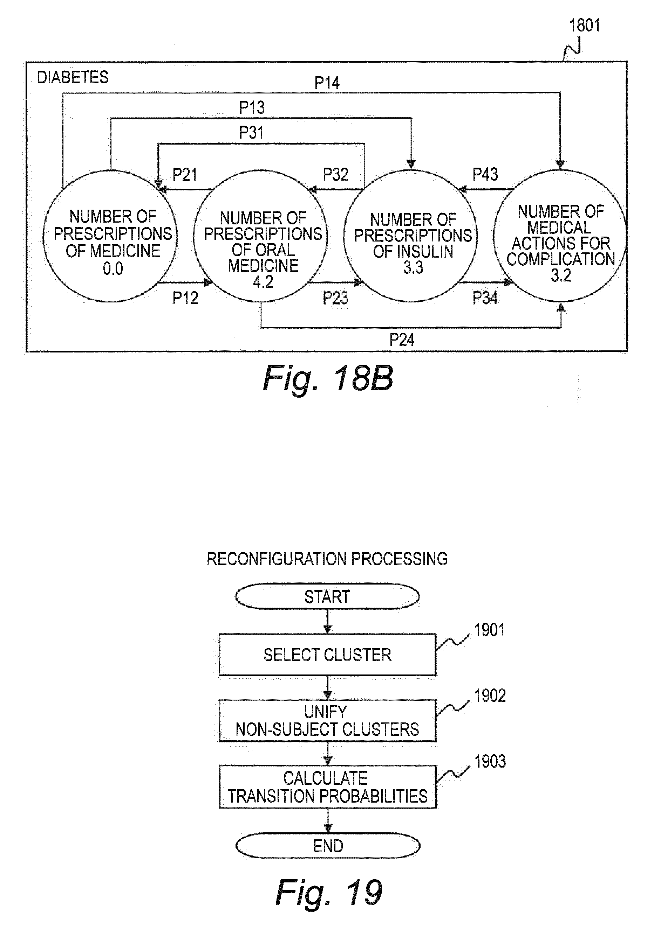

In Characterizing item selection step 1804, an item to be used for the characterization is selected. On this occasion, an item candidate to be used for the characterization may be manually determined in advance. On this occasion, a predetermined number of items highest in the deviation rate may be selected out of item candidates to be used for the characterization, and screen data for displaying the selected items and values thereof on the output unit 103 may be generated. FIG. 18B is a diagram illustrating a display example of the selected items and the values thereof. In a display example 1801, *Pij* represents the transition probability from a cluster *i* to a cluster *j*.

A description is now given of a method for the reconfiguration. The model generated by the state transition model generation unit 108 includes a large number of clusters, and such a problem that a calculation amount for the estimation is large in this state, the model is complex, the display is complicated, and understanding is difficult exists. Therefore, the generated model is reconfigured into a compact model, thereby modeling a state and a transition currently focused on.

FIG. 19 is a flowchart of reconfiguration processing.

First, in Cluster selection step 1901, based on an item specified as a subject to focusing, a cluster characterizing an item is selected. The items subject to focusing are such as the number of prescriptions of the insulin, the number of prescriptions of an oral antidiabetic, and the blood sugar level if the diabetes is focused on. First, a cluster having a value equal to or more than a predetermined value in the deviation rate in these items is selected. Then, clusters high in the transition probability (equal to or more than a predetermined value in the transition probability) from the cluster are selected.

Then, in Non-subject cluster unification step 1902, the clusters which are not selected are unified into one cluster. The selected clusters are set to *R1, R2, ... , RL,* and the clusters which are not selected are summarized into one cluster *O*. In other words, insured persons who do not belong to *R1, R2, ... , RL* are set to belong to the cluster *O*, and the model is constituted by the *L*+1 clusters.

In Transition probability calculation step 1903, a transition probability between each pair of clusters out of *L*+1 clusters constituting the model is calculated. A transition probability between the clusters *Ri* and *Rj* is the same as the transition probability *pij* between the original clusters generated by the state transition model generation unit 108. The transition probability from the cluster *R1* to the cluster *O* is 1-∑p1j. The summation is carried out for *j* from 1 to *L.* Conversely, the transition probability from the cluster *O* to the cluster *R1* is 1-∑pj1. The summation is carried out for *j* from 1 to *L.* As a result, the clusters and the transition probabilities between the clusters in the reconfigured model are generated. Moreover, the clusters are characterized by using the same method as that described before.

The state transition/medical cost estimation unit 114 uses the model generated by the state transition model generation unit 108 or the model reconfigured by the state characterization/reconfiguration unit 113 to estimate the state of disease and the medical cost in the next year from information in this year on each of insured persons subject to analysis specified by the healthcare guidance support unit 115. The state transition/medical cost estimation unit 114 stores the estimated states of the diseases and medical costs in an estimated result storage unit 122.

First, a description is given of a method for the state transition estimation, namely, a method of estimating the state in the next year from the information in this year on each of the insured persons subject to an analysis specified by the healthcare guidance support unit 115. First, the information in this year on each of the insured persons subject to the analysis is converted to formatted information in a form illustrated in FIG. 15 or 16A. Then, the similarities between the clusters $C1, ... , CK$ and all the insured persons are calculated based on the converted formatted information. A cluster $Ci$ highest in similarity is considered as the state in this year of the insured person subject to the analysis. The transition probability to each of the states in the next year can be calculated by the state transition probability stored in the state transition probability storage unit 121.

A description is now given of processing for the medical cost estimation. First, a cluster $Ci$ to which a insured person subject to the analysis belongs is specified based on the information of the insured person in this year by using the same method as in the state transition estimation. Moreover, the medical cost of each of clusters is calculated. The medical cost of the cluster $Cj$ is an average medical cost $M(j)$ of insured persons belonging to $Cj$. If the transition probability from the cluster $Ci$ to the cluster $Cj$ is represented as $P(j|i)$, $P(j|i)$ is such a probability that the insured person subject to the analysis is in the state of the cluster $Cj$ in the next year. Therefore, the expected value of the medical cost (estimated medical cost) in the next year is calculated by using $\sum P(j|i)M(j)$.

The healthcare guidance support unit 115 provides a function of supporting prevention of the disease onset of a disease in the future, and reduction of the medical cost.

A health insurance business operator wants to select subject people high in a prevention effect by means of the healthcare guidance by priority within the budget, and to provide a guidance appropriate for each of the subject people. A plurality of healthcare guidance services (such as a healthcare guidance service 1 and a healthcare guidance service 2) which the health insurance business operator can provide exist. For example, the healthcare guidance service 1 is such a guidance as to reduce mainly the BMI value, and the healthcare guidance service 2 is such a guidance to reduce the cholesterol level.

A description is now given of processing by a support function for a health insurance business operator.

FIG. 17A is a flowchart of the processing by the support function for a health insurance business operator.

First, in Subject disease setting step 1701, a disease subject to the processing is set. For example, if diabetes, dyslipidemia, and hypertension, which are the three major lifestyle-related diseases are considered as the subject, the state characterization/reconfiguration unit 113 reconstructs the model by using items of the medical action, items of the health checkup, and items of the medical inquiry corresponding to diabetes, dyslipidemia, and hypertension out of the items of the healthcare cost formatted information. If all the diseases are considered as the subject, the model generated by the state transition model generation unit 108 is used.

Then, in Healthcare guidance service setting step 1702, types of the healthcare guidance service and an estimated effect of each of the healthcare guidance services are set. For example, the estimated effect by the healthcare guidance service 1 is a weight loss by 5 kg, for example.

Then, in Healthcare guidance effect estimation step 1703, medical cost reduction effects are estimated for all combinations of each of the healthcare guidance services and each of the healthcare guidance subject candidates. First, a description is given of a calculation method for the medical cost reduction effect for a combination of the healthcare guidance service 1 and a healthcare guidance subject candidate 1.

First, a medical cost for the next year of the healthcare guidance subject candidate 1 if a healthcare guidance service is not provided is estimated. A state of the node corresponding to the items in this year is set based on values of the healthcare cost, health checkup, and medical inquiry of the healthcare guidance subject candidate 1 in this year, and the state transition/medical cost estimation unit 114 estimates a medical cost ($M1$). Then, a test value improved by the healthcare guidance service is set to the value of the healthcare guidance subject candidate 1 in this year, and the state transition/medical cost estimation unit 114 estimates a medical cost ($M2$) in the next year. $M1$ is the estimated medical cost without the healthcare guidance, and $M2$ is the estimated medical cost with the healthcare guidance. If a cost required for the healthcare guidance is $M3$, a medical-cost-reduction cost-effectiveness can be calculated as $E=M1-M2-M3$. This processing is carried out for all the combinations between each of the healthcare guidance services and each of the healthcare guidance subject candidates, thereby calculating pieces of the medical-cost-reduction cost-effectiveness E.

Then, in Healthcare guidance content planning step 1704, a combination highest in the medical-cost-reduction cost-effectiveness is selected out of the combinations of each of the healthcare guidance services and each of the healthcare guidance subject candidates. Then, the selected healthcare guidance subject candidate is set as "selected".

Then, a combination highest in the medical-cost-reduction cost-effectiveness is selected out of combinations of each of the healthcare guidance services and each of the healthcare guidance subject candidates, which have not been selected. Then, the selected healthcare guidance subject candidate is set as "selected". The combinations between each of the healthcare guidance services and each of the healthcare guidance subject candidates are selected in a descending order of the effect in this way. Finally, combinations high in the effect are selected within a budget of the healthcare guidance, thereby setting healthcare guidance subject people and healthcare guidance contents.

In Effect estimation step 1705, pieces of the medical-cost-reduction cost-effectiveness of the selected combinations in Healthcare guidance content planning step 1704 are summed, and a value acquired by subtracting the healthcare guidance cost from the medical cost reduction effect is output as the effect.

A description is now given of processing by a support function for a responsible person and a subject person.

FIG. 17B is a flowchart of the processing by the support function for a responsible person and a subject person.

First, in Subject disease setting step 1701, a disease subject to the processing is set. For example, if diabetes, dyslipidemia, and hypertension, which are the three major lifestyle-related diseases are considered as the subject, the state characterization/reconfiguration unit 113 reconstructs the model by using items of the medical action, items of the health checkup, and items of the medical inquiry corresponding to diabetes, dyslipidemia, and hypertension out of the items of the healthcare cost formatted information. If all the diseases are considered as the subject, the model generated by the state transition model generation unit 108 is used.

A description is now given of another example of the processing by Subject disease setting step 1701. A disease whose processing is requested by a subject person or a responsible person is selected. In other words, an item corresponding to a certain medical action is selected. Then, dependencies of the item on all the other items are calculated. Then, items equal to or more than a certain value in dependency on the selected item are extracted, and a model reconstructed by the state characterization/reconfiguration unit 113 based on the selected item and a list of the extracted items is used. On this occasion, the above-mentioned similarity between items may be used as the dependency.

In a disease onset probability calculation step 1706, the state transition/medical cost estimation unit 114 estimates disease state transition probabilities and the medical costs of the respective diseases in the next year while the states of all the nodes are not set yet. The disease onset probability of each of the diseases is acquired as a probability of a case where the number of prescriptions for a node relating to a medical action corresponding to the disease in the next year is equal to or more than 1. This probability can be considered as an average disease onset probability of the disease. Then, a state of the node corresponding to the item in this year is set based on values of the healthcare cost, the health checkup, and medical inquiry of the subject person in this year, and the state transition/medical cost estimation unit 114 estimates a disease state transition probability and a medical cost of each of the diseases in the next year. On this occasion, the disease onset probability of each of the diseases is the disease onset probability of the disease of the subject person. Thus, a ratio of a risk of developing disease of the subject person with respect to an average is calculated by dividing the disease onset probability of the disease of the subject person by an average disease onset probability of the disease for each of the diseases.

In High risk disease presentation step 1707, diseases having a risk of developing disease higher than the average by a predetermined threshold or more and risks thereof are presented. As a result, the subject person and the person responsible for the healthcare guidance can know the disease risks of the subject person.

In Improvement item presentation step 1708, test values each having a dependency equal to more than a certain value on a medical action node corresponding to the high risk disease calculated in High risk disease presentation step 1707 are presented. On this occasion, the above-mentioned similarity between items may be used as the dependency.

Then, in Target value user input step 1709, a user is prompted to input an improvement target value (such as a target value of the weight) for each of the test items presented in Improvement item presentation step 1708.

Finally, in Effect estimation step 1710, the value of each of the test items is updated by each of the target values input in Target value user input step 1709, and a disease onset probability of developing disease of each of the diseases after the target is attained is estimated by the same method as that in Step 1706, thereby presenting changes in the risks of developing disease. The user can recognize the changes in the risks of developing disease to set improvement targets, or to use the changes for self-management.

As a result of the processing, the healthcare guidance support effective for the medical cost reduction can be carried out.

The clustering is carried out by calculating the similarities between insured persons based on the healthcare cost information and the health checkup information, and classifying people into clusters each in a similar disease state based on the similarities as described above in the medical data analysis system according to the second embodiment. Therefore, insured persons in a similar state are clustered, and the state of the insured persons can be represented by the cluster.

Moreover, the state of the cluster is characterized by an average of states of the insured persons belonging to the cluster. Therefore, the state and the severity of a disease can be represented by the cluster. For example, the state of the cluster is represented by averages of the BMI value, the blood sugar level, the number of prescriptions of a clinical action, and the medical cost.

Moreover, the cluster is characterized by an item large in separation from a population, and hence the cluster can be automatically characterized.

Then, the transition between states (state and severity of the disease) is modeled by the transition probability between clusters. One insured person in a certain year belongs to any one of clusters, and one state of the disease corresponds to one cluster. Thus, a cluster to which a person belonging to the cluster belongs next year can be represented by the transition probability. Therefore, how a future transition occurs can be represented in an intuitively easy to understand manner as a transition between clusters.

Moreover, how many people are in what state can be easily recognized by the number of insured persons belonging to each cluster. Moreover, a difference in characteristic between groups can be recognized by comparing in a ratio of numbers of insured persons belonging to respective clusters between the groups. Differences in a disease rate, a ratio of serious case, and a state of increase in severity of diabetes can be recognized by comparing a group having a value equal to or less than a predetermined value in the blood sugar level and a group having a value more than the predetermined value in the blood sugar level with each other.

This model enables an efficient use of the healthcare cost information and the health checkup information including a large number of items, thereby representing a large number of diseases and states thereof. This model enables accurate and precise estimation of the future state transition and the medical cost of a disease.

Then, a healthcare guidance can be efficiently and effectively supported by selecting healthcare guidance subject people and healthcare guidance contents based on the estimation result.

Moreover, a cluster to which a person subject to the analysis belongs is determined based on the similarity between the insured persons, and, thus, an unknown subject person can be clustered.

Moreover, the similarity between insured persons is calculated by using an item acquired by unifying a plurality of items of the healthcare cost information, and the similarity can thus be highly precisely calculated.

Moreover, the similarity between insured persons is calculated by using the difference in position of the insured persons with respect to a population, and the similarity can thus be calculated using the same scale even between different items.

Moreover, the medical-cost-reduction cost-effectiveness is calculated as $M1-M2-M3$ ($M1$: estimated medical cost without a healthcare guidance, $M2$: estimated medical cost with the healthcare guidance, and $M3$: cost required for the healthcare guidance), and the medical-cost-reduction cost-effectiveness can thus be accurately estimated.

Moreover, the risk of developing disease for an improvement target value is calculated by using the transition probability between clusters, and the risk reduction effect by the improvement can thus be displayed in an easy-to-understand manner.

Moreover, a transition probability of at least one of diabetes, hypertension, or dyslipidemia is selected, and the healthcare guidance can thus be provided for diabetes, dyslipidemia, and hypertension, which are the three major lifestyle-related diseases.

This invention is not limited to the above-described embodiments but includes various modifications. The above-described embodiments are explained in details for better understanding of this invention and are not limited to those including all the configurations described above. A part of the configuration of one embodiment may be replaced with that of another embodiment; the configuration of one embodiment may be incorporated to the configuration of another embodiment. A part of the configuration of each embodiment may be added, deleted, or replaced by that of a different configuration.

The above-described configurations, functions, processing modules, and processing means, for all or a part of them, may be implemented by hardware: for example, by designing an integrated circuit. The above-described configurations and functions may be implemented by software, which means that a processor interprets and executes programs providing the functions. The information of programs, tables, and files to implement the functions may be stored in a storage device such as a memory, a hard disk drive, or an SSD (a Solid State Drive), or a storage medium such as an IC card, or an SD card. The drawings shows control lines and information lines as considered necessary for explanation but do not show all control lines or information lines in the products. It can be considered that almost of all components are actually interconnected.

**Claims**

1. An analysis system, comprising:

   a processor configured to execute a program;
   a memory configured to store the program,

the analysis system executing the program to analyze data,

the analysis system being capable of making access to a storage apparatus configured to store similarity information between data including a similarity between instances;

a data mapping unit configured to control the processor to set an attractive force and a repulsive force acting between the instances based on the similarity information between data, and arrange the instances in a vector space having a certain number of dimensions so that an energy caused by the attractive force and the repulsive force is less than a threshold defined in advance; and

a clustering unit configured to classify the instances arranged in the vector space,

wherein the data mapping unit is further configured to virtually add one dimension to the vector space, add a minute fluctuation to coordinates of the instances in a direction of the added dimension, and calculate a minimum number of dimensions of the vector space where the instances are stable with respect to the minute fluctuation.

2. An analysis system, comprising:

a processor configured to execute a program;

a memory configured to store the program,

the analysis system executing the program to analyze medical data,

the analysis system being capable of making access to a database configured to store medical information including an injury and illness name of a insured person and a medical action provided for the insured person, cost information on the medical action, and health checkup information including a test value acquired by a health checkup for the insured person;

a insured person arrangement coordinate generation unit configured to control the processor to calculate a similarity between insured persons based on the medical information and the health checkup information, and arrange the insured persons in a vector space having the same number of dimensions based on the calculated similarity;

a clustering unit configured to control the processor to classify the insured persons into clusters based on a coordinate at which each of the insured persons is arranged, and configure a pathologic transition model by using the classified clusters;

a pathologic transition probability calculation unit configured to control the processor to calculate a transition probability between the classified clusters;

a state characterization/reconfiguration unit configured to control the processor to reconfigure the pathologic transition model based on a cluster **characterized by** a specified item and a cluster determined by the characterized cluster and the transition probability;

a pathologic transition/medical cost estimation unit configured to control the processor to estimate, based on the cost information on the medical action and based on one of the configured pathologic transition model or the reconfigured pathologic transition model, future state and medical cost of a disease; and

a health guidance support unit configured to control the processor to select, based on the estimated future state and the estimated medical cost of the disease, a person subject to a health guidance and a content of the health guidance,

wherein the insured person arrangement coordinate generation unit is further configured to:

arrange the insured persons in a vector space having a certain number of dimensions so that an energy caused by an attractive force and a repulsive force acting between the insured persons set based on the similarity is less than a threshold defined in advance;

virtually add one dimension to the vector space having the certain number of dimensions;

add a minute fluctuation to coordinates of the insured persons in a direction of the added dimension;

calculate a minimum number of dimensions of the vector space where the insured persons are stable with respect to the minute fluctuation; and

arrange the insured persons in a vector space having the calculated number of dimensions.

3. The analysis system according to claim 2, wherein the health guidance support unit is further configured to:

identify a cluster to which a insured person high in similarity to a insured person subject to analysis belongs as a current state of the insured person subject to analysis; and

calculate, based on a probability of transition from the identified cluster to another cluster, a probability that the insured person subject to analysis belongs to the another cluster.

4. The analysis system according to claim 2, wherein the health guidance support unit is further configured to:

replace the health checkup information on a insured person subject to analysis by an estimated value after reception of the health guidance, and then identify a cluster to which a insured person high in similarity to the insured person subject to analysis belongs; and

calculate, based on a probability of transition from the identified cluster to another cluster, a probability that the insured person subject to analysis belongs to the another cluster after the reception of the health guidance.

**5.** The analysis system according to claim 2, wherein the state characterization/reconfiguration unit is further configured to:

characterize the cluster by means of values acquired by statistically processing values of respective items of the insured person belonging to the cluster; and

generate, for an item having a statistically processed value large in separation from an average of insured persons, data for displaying the item characterizing the cluster and the statistically processed value.

**6.** The analysis system according to claim 2, wherein the insured person arrangement coordinate generation unit is further configured to use an item acquired by unifying a plurality of items in the medical information to calculate the similarity between the insured persons.

**7.** The analysis system according to claim 2, further comprising a data formatting unit configured to control the processor to acquire a number of times of the medical action and a cost of the medical action from the medical information, to acquire the test value from the health checkup information, generate formatted information by summarizing the acquired information for each of the insured persons and for each predetermined period, and store the generated formatted information in the database,

wherein the insured person arrangement coordinate generation unit is further configured to calculate, for a certain item of the formatted information, the similarity between a first insured person and a second insured person by using a difference between a ratio of people having a value equal to or less than a value of the first insured person and a ratio of people having a value equal to or less than a value of the second insured person.

**8.** The analysis system according to claim 2, wherein the health guidance support unit is further configured to:

calculate an estimated effect by subtracting a second estimated medical cost acquired by replacing the test value of the insured person by a test value after the reception of the health guidance and a cost of the health guidance from the first estimated medical cost of the insured person; and

select a combination of a insured person high in the calculated estimated effect and the health guidance as the person subject to the health guidance and the content of the health guidance.

**9.** The analysis system according to claim 2, wherein the health guidance support unit is further configured to:

calculate a first risk of developing disease based on a ratio of a transition probability of a patholigical state of the insured person to an average transition probability of the pathological state;

calculate a second risk of developing disease by causing of an input of an improvement target value of the test value, and using a transition probability of the disease state acquired by replacing the test value of the insured person by the improvement target value; and

compare the first risk of developing disease and the second risk of developing disease with each other, to thereby generate data of a risk reduction effect through improvement.

**10.** The analysis system according to claim 9, wherein the analysis system is configured to configure a pathologic transition model for at least one of diabetes, hypertension, or dyslipidemia.

**11.** A health business support method for supporting a health guidance by using a computer including a processor for executing a program, and a memory for storing the program, the computer being capable of making access to a database for storing medical information including an injury and illness name of a insured person and a medical action provided for the insured person, cost information on the medical action, and health checkup information including a test value acquired by a health checkup for the insured person,

the health business support method including:

a insured person arrangement coordinate generation step of calculating, by the processor, a similarity between insured persons based on the medical information and the health checkup information, and arranging the insured persons in a vector space having the same number of dimensions based on the calculated similarity;

a clustering step of generating, by the processor, based on a coordinate at which each of the insured persons is arranged, a cluster into which the insured person is classified, and configuring a pathologic transition model by using generated clusters;

a state characterization/reconfiguration step of **characterizing, by** the processor, the cluster by means of a characteristic of the insured person belonging to the cluster, calculating, based on the medical information and the health checkup information, a transition probability between the generated clusters, and reconfiguring, based on the transition probability, a pathologic transition model configured by clusters **characterized by** a specified item;

a pathologic transition/medical cost estimation step of estimating, by the processor, based on information including a cost of the medical action, and one of the configured pathologic transition model or the reconfigured pathologic transition model, future state and medical cost of a disease; and

a health guidance support step of selecting, by the processor, based on the estimated state and the estimated medical cost of the disease, a person subject to a health guidance and a content of the health guidance,

wherein the clustering step including steps of:

setting an attractive force and a repulsive force acting between the insured persons based on the similarity between the insured persons;

arranging the insured persons in a vector space having a certain number of dimensions so that an energy caused by the attractive force and the repulsive force is less than a threshold defined in advance;

virtually adding one dimension to the vector space having the certain number of dimensions;

adding a minute fluctuation to coordinates of the insured persons in a direction of the added dimension;

calculating a minimum number of dimensions of the vector space where the insured persons are stable with respect to the minute fluctuation; and

arranging the insured persons in a vector space having the calculated number of dimensions.

Fig. 1

Fig. 2

SIMILARITY INFORMATION BETWEE DATA 301

|     | D1 | D2 | D3 | ... |
| --- | --- | --- | --- | --- |
| D1 | - | 0.53 | 0.92 | ... |
| D2 | - | - | 0.13 | ... |
| D3 | - | - | - |  |
| ... | - | - | - | - |

Fig. 3

GRAPH G =(V,E)

SET INITIAL COORDINATES — 401

SET ATTRACTIVE FORCE/
REPULSIVE FORCE — 402

COORDINATE
409 ARRANGEMENT
LOOP

SELECT SAMPLE — 403

COORDINATE
CORRECTION
LOOP
(1 CYCLE) — 410

CALCULATE FORCE — 404

CORRECT COORDINATE — 405

DOES NODE NOT
YET CORRECTED EXIST? — 406

YES

NO

CONVERGENCE TEST — 407

NO

YES

VISUALIZE — 408

*Fig. 4*

## SIMILARITY INFORMATION BETWEEN DATA

*Fig. 5*

HEALTHCARE COST BASIC INFORMATION

| SEARCH NUMBER | HEALTH INSURANCE INSURED PERSON ID | SEX | AGE | YEAR/MONTH OF CLINICAL ACTION | TOTAL POINT | ... |
|---|---|---|---|---|---|---|
| 11 | K0001 | MALE | 40 | 2004/06 | GT11 | |
| 12 | K0001 | MALE | 40 | 2004/07 | GT12 | |
| 13 | K0001 | MALE | 40 | 2004/08 | GT13 | |
| 21 | K0002 | MALE | 45 | 2004/04 | GT21 | |
| 22 | K0002 | MALE | 45 | 2004/09 | GT22 | |
| 31 | K0003 | MALE | 50 | 2004/06 | GT31 | |
| 32 | K0003 | MALE | 50 | 2004/12 | GT32 | |
| 41 | K0004 | FEMALE | 50 | 2005/01 | GT41 | |
| ... | | | | | | |

*Fig. 6*

HEALTH CHECKUP INFORMATION

| HEALTH INSURANCE INSURED PERSON ID | DATE OF HEALTH CHECKUP | BMI | ABDOMINAL CIRCUM-FERENCE | FASTING BLOOD SUGAR | SYSTOLIC BLOOD PRESSURE | NEUTRAL FAT | ... |
|---|---|---|---|---|---|---|---|
| K0001 | 2004/05/09 | 25 | 87 | 105 | 130 | 150 | |
| K0001 | 2005/05/15 | 24 | 84 | 100 | 125 | 130 | |
| K0002 | 2004/05/13 | 22 | 70 | 110 | 119 | 90 | |
| K0002 | 2005/05/20 | 21 | 70 | 108 | 119 | 90 | |
| K0003 | 2004/06/05 | 25 | 87 | 110 | 120 | 150 | |
| K0003 | 2005/06/10 | 23 | 87 | 111 | 122 | 155 | |
| K0004 | 2004/02/10 | 20 | 92 | 90 | - | 140 | |
| K0004 | 2005/02/20 | 20 | 90 | 90 | 120 | 130 | |
| ... | | | | | | | |

*Fig. 7*

MEDICAL INQUIRY INFORMATION

| HEALTH INSURANCE INSURED PERSON ID | DATE OF MEDICAL INQUIRY | SMOKING | DRINKING | WALKING | ... |
|---|---|---|---|---|---|
| K0001 | 2004/05/09 | NONE | 100 | 20 | |
| K0001 | 2005/05/15 | NONE | NONE | 40 | |
| K0002 | 2004/05/13 | 10 | 200 | 10 | |
| K0002 | 2005/05/20 | 7 | NONE | 60 | |
| K0003 | 2004/06/05 | NONE | 50 | - | |
| K0003 | 2005/06/10 | NONE | SLIGHT | 20 | |
| K0004 | 2004/02/10 | 15 | NONE | 30 | |
| K0004 | 2005/02/20 | 3 | NONE | 10 | |
| ... | | | | | |

603  802  803  804  805  801

## Fig. 8

INJURY AND ILLNESS NAME INFORMATION

| 602 | 902 | 903 | 901 |
|---|---|---|---|
| SEARCH NUMBER | INJURY AND ILLNESS CODE | INJURY AND ILLNESS NAME | ... |
| 11 | 10 | DIABETES | |
| 11 | 20 | HYPERTENSION | |
| 12 | 30 | BONE FRACTURE | |
| 13 | 10 | DIABETES | |
| 13 | 20 | HYPERTENSION | |
| 13 | 40 | GOUT | |
| 21 | 10 | DIABETES | |
| 21 | 11 | DIABETIC NEPHROPATHY | |
| 22 | 10 | DIABETES | |
| 22 | 11 | DIABETIC NEPHROPATHY | |
| 22 | 20 | HYPERTENSION | |
| 31 | 10 | DIABETES | |
| 32 | 10 | DIABETES | |
| 41 | 50 | INFLUENZA | |
| ... | | | |

Fig. 9

INJURY AND ILLNESS NAME CLASSIFICATION INFORMATION

| INJURY AND ILLNESS CLASSIFICATION | INJURY AND ILLNESS CODE | INJURY AND ILLNESS NAME | COMPLICATION |
|---|---|---|---|
| DIABETES | 10 | DIABETES | ABSENT |
| DIABETES | 11 | DIABETIC NEPHROPATHY | PRESENT |
| DIABETES | 12 | DIABETIC RETINOPATHY | PRESENT |
| ··· | | | |
| HYPER-TENSION | 20 | HYPERTENSION | ABSENT |
| ··· | | | |

*Fig. 10*

CLINICAL ACTION INFORMATION

| SEARCH NUMBER | CLINICAL ACTION CODE | CLINICAL ACTION NAME | CLINICAL ACTION POINT | ... |
|---|---|---|---|---|
| 11 | 1000 | CLINICAL ACTION A | ST1000 | |
| 11 | 2000 | CLINICAL ACTION C | ST2000 | |
| 12 | 3000 | CLINICAL ACTION F | ST3000 | |
| 13 | 1000 | CLINICAL ACTION A | ST1000 | |
| 13 | 2000 | CLINICAL ACTION C | ST2000 | |
| 21 | 1000 | CLINICAL ACTION A | ST1000 | |
| 21 | 1100 | CLINICAL ACTION B | ST1100 | |
| 22 | 1000 | CLINICAL ACTION A | ST1000 | |
| 22 | 1100 | CLINICAL ACTION B | ST1100 | |
| 22 | 2000 | CLINICAL ACTION C | ST2000 | |
| 31 | 1100 | CLINICAL ACTION A | ST1100 | |
| 32 | 1100 | CLINICAL ACTION A | ST1100 | |
| ... | | | | |

*Fig. 11*

CLINICAL ACTION CLASSIFICATION INFORMATION

| INJURY AND ILLNESS CLASSIFICATION | CLINICAL ACTION CODE | CLINICAL ACTION NAME |
|---|---|---|
| DIABETES | 1000 | CLINICAL ACTION A |
| DIABETES | 1100 | CLINICAL ACTION B |
| ... | | |
| HYPERTENSION | 2000 | CLINICAL ACTION C |
| ... | | |

*Fig. 12*

MEDICINE INFORMATION

| SEARCH NUMBER 602 | MEDICINE CODE 1302 | MEDICINE NAME 1303 | MEDICINE POINT 1304 | ... 1301 |
|---|---|---|---|---|
| 11 | 110 | ORAL ANTIDIABETIC A | ET110 | |
| 11 | 200 | HYPERTENSION ORAL MEDICINE A | ET200 | |
| 12 | 300 | ANODYNE A | ET300 | |
| 13 | 111 | ORAL ANTIDIABETIC B | ET111 | |
| 13 | 210 | HYPERTENSION ORAL MEDICINE B | ET210 | |
| 21 | 110 | ORAL ANTIDIABETIC A | ET110 | |
| 21 | 120 | INSULIN PREPARATION A | ET120 | |
| 22 | 110 | ORAL ANTIDIABETIC A | ET110 | |
| 22 | 120 | INSULIN PREPARATION A | ET120 | |
| 22 | 200 | HYPERTENSION ORAL MEDICINE A | ET200 | |
| 31 | 121 | INSULIN PREPARATION B | ET121 | |
| 31 | 111 | ORAL ANTIDIABETIC B | ET111 | |
| 32 | 121 | INSULIN PREPARATION B | ET121 | |
| ... | | | | |

*Fig. 13*

MEDICINE CLASSIFICATION INFORMATION

1002　　　　　　1302　　　　　　1303　　　1401

| INJURY AND ILLNESS CLASSIFICATION | MEDICINE CODE | MEDICINE NAME |
|---|---|---|
| DIABETES | 110 | ORAL ANTIDIABETIC A |
| DIABETES | 111 | ORAL ANTIDIABETIC B |
| DIABETES | 120 | INSULIN PREPARATION A |
| DIABETES | 121 | INSULIN PREPARATION B |
| • • • | | |
| HYPERTENSION | 200 | HYPERTENSION ORAL MEDICINE A |
| • • • | | |

*Fig. 14*

FORMATTED INFORMATION

| 603 HEALTH INSURANCE INSURED PERSON ID | 1502 DATA YEAR | 604 SEX | 605 AGE | 1503 INJURY AND ILLNESS CODE 10 | 1504 INJURY AND ILLNESS CODE 20 | ... | 1505 MEDICAL ACTION CODE | ... | 1506 MEDICINE CODE 110 | ... | 607 TOTAL POINT | ... |
|---|---|---|---|---|---|---|---|---|---|---|---|---|
| K0001 | 2004 | MALE | 40 | 2 | 2 | | 2 | | 1 | | GT11 | |
| K0002 | 2004 | MALE | 45 | 2 | 1 | | 2 | | 2 | | GT12 | |
| K0003 | 2004 | MALE | 50 | 2 | 0 | | 0 | | 0 | | GT13 | |
| K0004 | 2004 | FEMALE | 50 | 0 | 0 | | 0 | | 0 | | GT21 | |
| ... | | | | | | | | | | | | |

| 1508 BMI | 1509 ABDOMINAL CIRCUM-FERENCE | 1510 FASTING BLOOD SUGAR | 1511 SYSTOLIC BLOOD PRESSURE | 1512 NEUTRAL FAT | 1514 SMOKING | 1515 DRINKING | 1516 WALKING | ... |
|---|---|---|---|---|---|---|---|---|
| 25 | 87 | 105 | 130 | 150 | 0 | 100 | 20 | |
| 22 | 70 | 110 | 119 | 90 | 10 | 200 | 10 | |
| 25 | 87 | 110 | 120 | 150 | 0 | 50 | -1 | |
| 20 | 92 | 90 | -1 | 140 | 15 | 0 | 30 | |
| | | | | | | | | |

*Fig. 15*

FORMATTED INFORMATION

| 603 HEALTH INSURANCE INSURED PERSON ID | 1502 DATA YEAR | 604 SEX | 605 AGE | 1601 INJURY AND ILLNESS CODE 10, 20 | ... | 1505 CMEDICAL ACTION CODE 1000 | ... | 1506 MEDICINE CODE 110 | ... | 607 TOTAL POINT | ... |
|---|---|---|---|---|---|---|---|---|---|---|---|
| K0001 | 2004 | MALE | 40 | 4 | | 2 | | 1 | | GT11 | |
| K0002 | 2004 | MALE | 45 | 3 | | 2 | | 2 | | GT12 | |
| K0003 | 2004 | MALE | 50 | 2 | | 0 | | 0 | | GT13 | |
| K0004 | 2004 | FEMALE | 50 | 0 | | 0 | | 0 | | GT21 | |
| ... | | | | | | | | | | | |

| 1508 BMI | 1509 ABDOMINAL CIRCUM-FERENCE | 1510 FASTING BLOOD SUGAR | 1511 SYSTOLIC BLOOD PRESSURE | 1512 NEUTRAL FAT | 1514 SMOKING | 1515 DRINKING | 1516 WALKING | ... |
|---|---|---|---|---|---|---|---|---|
| 25 | 87 | 105 | 130 | 150 | 0 | 100 | 20 | |
| 22 | 70 | 110 | 119 | 90 | 10 | 200 | 10 | |
| 25 | 87 | 110 | 120 | 150 | 0 | 50 | -1 | |
| 20 | 92 | 90 | -1 | 140 | 15 | 0 | 30 | |
| | | | | | | | | |

*Fig. 16A*

ITEM UNIFICATION PROCESSING

```
        ┌─────────────┐
        │    START     │
        └─────────────┘
               │
               ▼
   ┌──────────────────────┐
   │  SELECT ITEMS SUBJECT │ ～ 1602
   │    TO UNIFICATION     │
   └──────────────────────┘
               │
               ▼
   ┌──────────────────────┐
   │  SUM NUMBERS OF TIMES │ ～ 1603
   │    OF PRESCRIPTION    │
   └──────────────────────┘
               │
               ▼
        ┌─────────────┐
        │    END       │
        └─────────────┘
```

## Fig. 16B

SUPPORT FUNCTION FOR
HEALTH INSURANCE BUSINESS OPERATOR

```
        ┌─────────────┐
        │    START     │
        └─────────────┘
               │
               ▼
   ┌──────────────────────┐
   │  SET SUBJECT DISEASE  │ ～ 1701
   └──────────────────────┘
               │
               ▼
   ┌──────────────────────┐
   │   SET HEALTHCARE      │ ～ 1702
   │  GUIDANCE SERVICE     │
   └──────────────────────┘
               │
               ▼
   ┌──────────────────────┐
   │ ESTIMATE HEALTHCARE   │ ～ 1703
   │  GUIDANCE EFFECT      │
   └──────────────────────┘
               │
               ▼
   ┌──────────────────────┐
   │   PLAN HEALTHCARE     │ ～ 1704
   │  GUIDANCE CONTENT     │
   └──────────────────────┘
               │
               ▼
   ┌──────────────────────┐
   │   ESTIMATE EFFECT     │ ～ 1705
   └──────────────────────┘
               │
               ▼
        ┌─────────────┐
        │    END       │
        └─────────────┘
```

## Fig. 17A

SUPPORT FUNCTION FOR
RESPONSIBLE PERSON AND SUBJECT PERSON

START

SET SUBJECT DISEASE — 1701

CALCULATE
DISEASE ONSET PROBABILITIES — 1706

PRESENT HIGH RISK
DISEASES — 1707

PRESENT ITEMS
TO BE IMPROVED — 1708

INPUT TARGET VALUES
BY USER — 1709

ESTIMATE EFFECTS — 1710

END

*Fig. 17B*

CLUSTER CHARACTERIZATION PROCESSING

START

CALCULATE STATISTICAL
AMOUNTS IN CLUSTER — 1802

CALCULATE
ABNORMAL ITEMS — 1803

SELECT ITEM TO BE USED
FOR CHARACTERIZATION — 1804

END

*Fig. 18A*

1801

DIABETES

P14

P13

P31

P21          P32          P43

| NUMBER OF PRESCRIPTIONS OF MEDICINE 0.0 | NUMBER OF PRESCRIPTIONS OF ORAL MEDICINE 4.2 | NUMBER OF PRESCRIPTIONS OF INSULIN 3.3 | NUMBER OF MEDICAL ACTIONS FOR COMPLICATION 3.2 |

P12          P23          P34

P24

*Fig. 18B*

RECONFIGURATION PROCESSING

START

SELECT CLUSTER — 1901

UNIFY NON-SUBJECT CLUSTERS — 1902

CALCULATE TRANSITION PROBABILITIES — 1903

END

*Fig. 19*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013104659 A

- JP 2012128670 A

**Non-patent literature cited in the description**

- **Y. F. HU.** Efficient, High-Quality Force-Directed Graph Drawing. *The Mathematica Journal,* 2006, vol. 10 (1), 37-71